(19) Europäisches Patentamt
European Patent Office
Office européen des brevets

(11) EP 2 305 810 A1

(12) **EUROPEAN PATENT APPLICATION**

(43) Date of publication:
**06.04.2011 Bulletin 2011/14**

(21) Application number: **09172129.0**

(22) Date of filing: **02.10.2009**

(51) Int Cl.:
***C12N 15/11*** *(2006.01)* ***A61K 31/7088*** *(2006.01)*
***A61P 19/04*** *(2006.01)* *A61P 9/00* *(2006.01)*

(84) Designated Contracting States:
**AT BE BG CH CY CZ DE DK EE ES FI FR GB GR HR HU IE IS IT LI LT LU LV MC MK MT NL NO PL PT RO SE SI SK SM TR**

(71) Applicant: **Technische Universität München**
**80333 München (DE)**

(72) Inventor: **The designation of the inventor has not yet been filed**

(74) Representative: **Heunemann, Dieter**
**Vossius & Partner**
**Siebertstrasse 4**
**81675 München (DE)**

(54) **miRNAs in the treatment of fibrosis**

(57) The present invention relates to nucleic acid molecules comprising at least ten contiguous bases having a sequence as shown in the sequence of miR-223 (SEQ ID NO: 16) for treating fibrosis. Also methods for treating fibrosis are provided.

Figure 1.

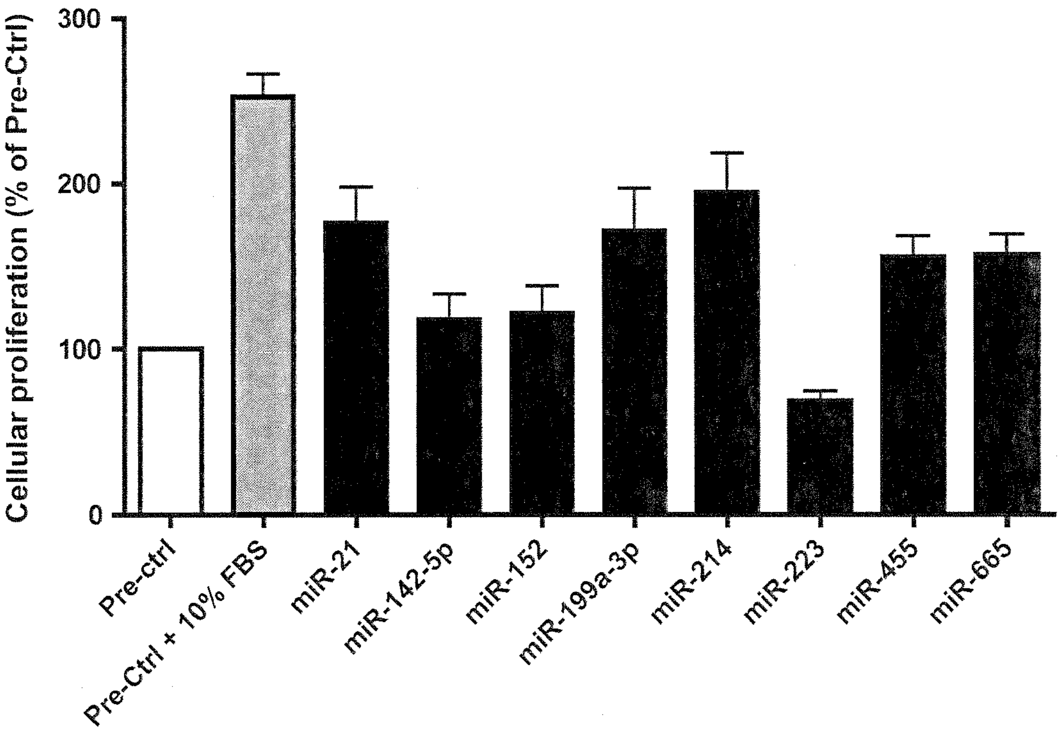


EP 2 305 810 A1

SEQUENCE LISTING

<110> Technische Universität München

<120> miRNAs in the treatment of fibrosis

<130> R2777 EP S3

<160> 27

<170> PatentIn version 3.5

<210> 1
<211> 72
<212> RNA
<213> homo sapiens

<400> 1
ugucggguag cuuaucagac ugauguugac uguugaaucu cauggcaaca ccagucgaug 60

ggcugucuga ca 72

<210> 2
<211> 87
<212> RNA
<213> homo sapiens

<400> 2
gacagugcag ucacccauaa aguagaaagc acuacuaaca gcacuggagg guguaguguu 60

uccuacuuua uggaugagug uacugug 87

<210> 3
<211> 87
<212> RNA
<213> homo sapiens

<400> 3
uguccccccc ggcccagguu cugugauaca cuccgacucg ggcucuggag cagucagugc 60

augacagaac uugggcccgg aaggacc 87

<210> 4
<211> 71
<212> RNA
<213> homo sapiens

<400> 4
gccaacccag uguucagacu accuguucag gaggcucuca auguguacag uagucugcac 60

auugguuagg c 71

<210> 5
<211> 110
<212> RNA
<213> homo sapiens

<400> 5
aggaagcuuc uggagauccu gcuccgucgc cccaguguuc agacuaccug uucaggacaa 60

ugccguugua caguagucug cacauugguu agacugggca agggagagca 110

<210> 6
<211> 110
<212> RNA
<213> homo sapiens

<400> 6
ggccuggcug gacagaguug ucauguqucu gccugucuac acuugcugug cagaacaucc 60

gcucaccugu acagcaggca cagacaggca gucacaugac aacccagccu 110

<210> 7
<211> 110
<212> RNA
<213> homo sapiens

<400> 7
ccuggccucc ugcagugcca cgcuccgugu auuugacaag cugaguugga cacuccaugu 60

gguagagugu caguuuguca aauaccccaa gugcggcaca ugcuuaccag 110

<210> 8
<211> 96
<212> RNA
<213> homo sapiens

<400> 8
ucccuggcgu gaggguaugu gccuuuggac uacaucgugg aagccagcac caugcagucc 60

augggcauau acacuugccu caaggccuau gucauc 96

<210> 9
<211> 72
<212> RNA
<213> homo sapiens

<400> 9
ucuccucgag ggucucugc cucuacccag gacucuuuca ugaccaggag gcugaggccc 60

cucacaggcg gc 72

<210> 10
<211> 22
<212> RNA
<213> homo sapiens

<400> 10
uagcuuauca gacugauguu ga 22

<210> 11
<211> 21
<212> RNA
<213> homo sapiens

<400> 11

cauaaaguag aaagcacuac u 21

<210> 12
<211> 21
<212> RNA
<213> homo sapiens

<400> 12
ucagugcaug acagaacuug g 21

<210> 13
<211> 22
<212> RNA
<213> homo sapiens

<400> 13
acaguagucu gcacauuggu ua 22

<210> 14
<211> 22
<212> RNA
<213> homo sapiens

<400> 14
acagcaggca cagacaggca gu 22

<210> 15
<211> 22
<212> RNA
<213> homo sapiens

<400> 15
ugucaguuug ucaaauaccc ca 22

<210> 16
<211> 21
<212> RNA
<213> homo sapiens

<400> 16
gcaguccaug ggcauauaca c 21

<210> 17
<211> 20
<212> RNA
<213> homo sapiens

<400> 17
accaggaggc ugaggccccu 20

<210> 18
<211> 7
<212> RNA
<213> homo sapiens

<400> 18

gucaguu 7

<210> 19
<211> 1272
<212> DNA
<213> homo sapiens

<400> 19
tccacctaca acaagaattt cttaagcttt cttttatttg catgagagag ccactaccaa 60

ggcatgtttt gttatgctga aactgggctg ctgcatactg ctaaatggca cctctgggat 120

tggcctacct ggggatttct tggtttgtga aaacaggaga ggagaaatat ctcatacaag 180

tgaaaggata ctggagagag aaattaccca tttctaaaaa aaaaccacac tctgtcgtat 240

ctgtgttaat gttttctagc atgtactctg gtttcaacag acacaaattt atatgttaac 300

ccagttttct tgccgttctg taagtgtttt attcttagtg tgattttttt ccattgggat 360

gtttttgatt gaacttgttc attttgtttt gcttgggagg aaaataaaca attttacttt 420

tttcctttag gagcattatg agcattatgt cagaatagaa tagaattggg gttcgatctt 480

aacaggccag aaatgcctgg gtttttttgg tttgtttttg ttttgttttt tttatcaaat 540

cctgcctgac tgtctgcttg ttttgcctac catcgtgaca tctccatggc tgtaccacct 600

tgtcgggtag cttatcagac tgatgttgac tgttgaatct catggcaaca ccagtcgatg 660

ggctgtctga cattttggta tctttcatct gaccatccat atccaatgtt ctcatttaaa 720

cattacccag catcattgtt tataatcaga aactctggtc cttctgtctg gtggcactta 780

gagtcttttg tgccataatg cagcagtatg gagggaggat tttatggaga aatggggata 840

gtcttcatga ccacaaataa ataaaggaaa actaagctgc attgtgggtt ttgaaaaggt 900

tattatactt cttaacaatt ctttttttca gggacttttc tagctgtatg actgttactt 960

gaccttcttt gaaaagcatt cccaaaatgc tctattttag atagattaac attaaccaac 1020

ataatttttt ttagatcgag tcagcataaa tttctaagtc agcctctagt cgtggttcat 1080

ctctttcacc tgcattttat ttggtgtttg tctgaagaaa ggaaagagga aagcaaatac 1140

gaattgtact atttgtacca aatctttggg atttcattggc aaataatttc agtgtggtgt 1200

attattaaat agaaaaaaaa aattttgttt cctaggttga aggtctaatt gatacgtttg 1260

acttatgatg ac 1272

<210> 20
<211> 1287
<212> DNA
<213> homo sapiens

<400> 20
gaggctggga attgaggccc tggtgtgttc aagaccttgg gctggtgggg aggccagggc 60

ctgaggccct gggggtcag gaccctgggc tgttggggag gctggggact taggccctgg 120

ggggtcagga tcttgggctg gtggggaggc tgaagggtgg ggactgaggc tctcggcagt 180

caggacctca cggttggcgt ggactggaga ccaagacctt ggcaggggag ctgtggctgc 240

ctcatttgga cgctggaggg tggctagcgt ggctggaagc ggccagccag gggttcacag 300

aactgaaggt gaggccccca gaggccctag tctctacctg agtgtctctg aaactggggg 360

gatggggtgg agcctttagg ggaagggaa gagggaactg aagaggaagt gggggaggga 420

ggtagaggag gcaagtctgg cgcccatgctg agtcaccgcc cacaaggccc agggcgggcc 480

ctcgggggc cctggcaggg ttgggggat cttaggaagc cacaaggagg gctgggggc 540

tcttggagca ggagtcagga ggcctgggca gcctgaagag tacacgccga cggacagaca 600

gacagtgcag tcacccataa agtagaaagc actactaaca gcactggagg gtgtagtgtt 660

tcctacttta tggatgagtg tactgtgggc ttcggagatc acgccactgc tgcccgcccgc 720

tgcccgccac catcttcctc ggcgctcggg gacctcgtgt gacaggtgag caccttacgg 780

cccctcccta ccctgcccag atgcctgaaa ggcctccatg gctttcctgc ccttcctggt 840

tccggacagc tggggaaacg ccacagcagc tcctctgctg ccctgcagtc tttgggggcg 900

ggcagggctg gacatgtgga accctgatgc agccgcagcg tcaaggacga ggaagggggtg 960

ggaagggatg gtacgtggag gggaatgggt ggtgggacca cggacccaat gctaataaag 1020

actggactgt gcttctcttt tgtctgagac tctgttgctg gggtggggag ctgaatgcgg 1080

gcggcaggca catggacagt atgggcgcac agaagggctg ggagggagaa cctctgtcgc 1140

tcctccagaa gatacgggtt tggccctgcc tatgagatca gaagagggct ctgggcctgg 1200

ccaggccaca gccggaggag agaaggccct gggccatctg cagaaagggg cagggatgta 1260

aggcatcctc tcaccttcta ccctagc 1287

<210> 21
<211> 1287
<212> DNA
<213> homo sapiens

<400> 21
gggcggcgc ggaggcgggg cggcggcggc cggcaggcgg cgggcggcgc tgtcagtgcg 60

aggcggcgag cggaatgcag cggcccgagg ctggccacgt ccgcacccgg gggagggggc 120

cgcggcggca ggccgggggc cggcgcccgc tgctcgagcc ggggagccct cggggctgcg 180

ggtacggagc gggcgggcgc ggggatcccg ggcggcggga gctgggcagg gacagagcag 240

ggttaggggg agcagccaac tcagaactcg cgcgcccggc ggctgggaac tttgtgtcac 300

ccctgactgg ccccaggacc cgggaggaaa agttcgtccc agcgccgacc ggagagcagg 360

cctgtcctga gtccgtcggc gcggggctga agttctgggt ccgtttggac tgggggtggc 420

gggtgagttg cgattccgcg gggagggtaa ggagtggcct gtccgtccca gactcggctc 480

ccatcaccgg tgggtgatgg gcatgcttct ggagtctacc cgggccaggg cctgggggcg 540
cgctgatagc gcaggtccag cccggccagg gatcagctgg aagaaggagg ctcggcccgc 600
tgtccccccc ggcccaggtt ctgtgataca ctccgactcg ggctctggag cagtcagtgc 660
atgacagaac ttgggcccgg aaggaccttc tgcacccaac gggcacagcg cccactcggg 720
gcctgcagtg gaacatctgc ctgggagtgc agtgggcacc tgggttggcc catgtggcac 780
agggctgggc agaaccagct gtggaccttg aggactgggg ttgctggggc ctgaggatgt 840
atcctagggc cagcagatcc catagacaca gggtgtccac ctcgagtcag gggccaaacg 900
gaactctcct ctccggtgtc agttgcagga accttccctc tacaccatca aggctgtttt 960
catcctagat aatgacgggc gccggctgct ggccaaggta acctctgacc ccacccatga 1020
gggacacagt tcccactcag ctgacactcc tgtctcagga gaaactccac tgtgatctct 1080
gtgactcagg gcagctcagc ttagacccct ccagaggtcc ttctcatcct cctgccttgt 1140
caaggtgctg ctccctgaaa tgaggggtct cctctatttc tactcatgtt ccctgaaatg 1200
tttccttatc atcctgctac tggccagttg gacctgggca tgggaaggtt ctgcctaatg 1260
ggtcagtgga gtgttgagcc tgggacc 1287

<210> 22
<211> 1271
<212> DNA
<213> homo sapiens

<400> 22
ctcccacctt tcggggaaga gttttggggg ctttagaagg ggctagggga ggggctctg 60
cagaagccct gccccggct cggacgtttg cccaagggcc tttgctgccc tgaccctatc 120
atatcacgcg gccggaagct tccagccagc agggccaagt aactgagtca caggccattc 180
cagctaatgc cacatctgga actgtttaca gtgcgattcc gccgagaaat cagtggccgc 240
cttcctggtg ctgccggcac gcacgcgtgc gcgcgcgcgc acacacacac acacacgtgt 300
gtgtgcgccc ctcctcccca cccccgaccc ccaaagagtc agacattcct cctgagccca 360
gaagccacga tcccaaaccc tgcctcctgc tccgcctccc ccactcttta ggatttcctg 420
aaaacccaga actttctcca gatgcgagcc ggccccagcc ttgccacgtc agaaggcaca 480
gagcggatcg tctcgggaag agtggtggtt tccttggctg ctcagaggtg ctgaggcacg 540
gcctggcctg gtggccccag cgtctgcctg gggggttctg caggatggat agccggcccc 600
gccaacccag tgttcagact acctgttcag gaggctctca atgtgtacag tagtctgcac 660
atcggttagg ctgggcttgg gtgagcggct cgtcgagaca ggccccccaa actcgccggc 720
aggtgagtgt cattttccac caccccgttt cccactgtgg cagagcctcg catagaagat 780
tcgagggcct gggtgggaga agaggcactg gggaaaggca gagggagccc cgaggccggc 840

cgaggggttg gaggccctgg ccctatcagt ctgccatccc caccccatgg ctgtaaatgt 900
cttgtttatt ttttaaataa agagatattg atgtcttgtg tctcactgag gcatctaaga 960
gggtggcctc ctcctcaggg gtggctgcca cgtgtgtgac tccaagggtc cctgctggga 1020
tcctaacttg ggtggtggcg gggtcagct tccatggctg cccccctcctg aatggagcct 1080
gctttgtgtc tggggctcac acagggcccc tttcacaggt cggctgtttc tcattacggc 1140
cggcgcccgg cccttctccc actgctgctc cagactccag tgtggggctc cacggcaggg 1200
tcgggggctc tggctttcaa ctctcagatg agaaaaacac gaagggcccc tcacctgaag 1260
gaagaggaga t 1271

<210> 23
<211> 1310
<212> DNA
<213> homo sapiens

<400> 23
gctcaaaaag gtttccaaat gagctctgcc ttccagtgtt caactttctg tttactattt 60
catctaccct ctatcttcaa attacttctc tagcctgggt cagacagtaa atttgtactt 120
ctacactaac tcactttcag ttggccacag ccgaactgta catcttgact cttttttttt 180
tacagctttt ccatactggg gcccacttcc tgcccagttg aggaaaaaaa tctggcctga 240
agatgaaatg actgcttaac gttacactaa aacatctggt accattttat gcacagaccc 300
atgtctcaga gcaggcgatt ctagcggtct ctccagcggc acagcgcata cccaaacctc 360
cccagggtga catcatccca tatatggact ctccagccca gccctccccc ttttcctggt 420
cctaaattca ttgccagttc cccagtctgc agcaaatgtg ccacgtcaag actggaaatc 480
acagcccttg agtgtgtctc agtcagcgca gcagaattca gggggaaag ctgaatgcaa 540
cccctggtgc aggaagggag gcttttcctg aggaccggga gaggatttta agtacataga 600
aggaagcttc tggagatcct gctccgtcgc cccagtgttc agactacctg ttcaggacaa 660
tgccgttgta cagtagtctg cacattggtt agactgggca agggagagca acgccatgga 720
ccgctgggga caaaatgggc tgtttccaac gagaagacat ttgtttgctc ctttttgaa 780
tctcatatcc agtgtttttc ttcacaggtt ttgcacacta gggaccaaga agccctcggg 840
gacactgctg agaaaagact gccgaaggga agaccagcgg gtaaggcctt cttgactgct 900
cacctggtct gtgaccaagg gaacctaggt ggggagccca tgatttgtgt taacatgcaa 960
gggaaacaga ggtacatgtg tgatgggtag agatgtattt aattttaaaa gagcaaaagt 1020
gtgtggtata tagcgattta ctgttttttt ttttttattt tctgaagatt gataattcaa 1080
gtgttttctt aaaggttttc ccacttcaaa atgaaatatg aaaaaactca ttccagacac 1140
ttaagaatag tttaatgagg ctacttttga attgtttaat aataattcaa agatttaaaa 1200

acttatatgg atgaaggctg tatgtataac ttttaacatc ccaggactga agtcattttt 1260

ccccaaagta aagaatactt gctggttttt aaatgctata tggatagtgc 1310

<210> 24
<211> 1310
<212> DNA
<213> homo sapiens

<400> 24
tttcacatat aattggtaaa aacatattcc cttgtaggat cctattatca gagcatcaaa 60

taccaaagtc agcaacttgg gttgccaaat aaattaggct cattaattat tttgaaaagg 120

gctgctgttt ccatgtaaat gataatttic ttttcatgg acaaggttat ggtacttatc 180

tactaaaaga aacatgcata aacatagttt ttatctttaa cttcagtatt aaaatatgca 240

gtttttttgca aagaggaaaa acatctggtt caattacgct gaactctgac tacatgtggg 300

ccagtaataa tatgaattgg acttaagaat aaaccttgtg tttaatctct ttttttcctt 360

aaaattttaa tgtgagtttt ctgttacgca aattatccat gttagcacat ttggaacaaa 420

tgtataaatg tactttctga ataaagtcaa aagtctaact tgttttttagt tccataatgt 480

tttaatgttt aattatattg tgtatttttc tccctttccc cttactctcc aaaatcacca 540

aatctggaaa acaggctgat tgtatctgtc tatgagcaaa ggaaacctga aggaaccaag 600

ggcctggctg gacagagttg tcatgtgtct gcctgtctac acttgctgtg cagaacatcc 660

gctcacctgt acagcaggca cagacaggca gtcacatgac aacccagcct caatgacaac 720

cagccattga aagaaagcag ccctcacacc atagcatcta caccaagagc tacaaccaaa 780

atgaggggct cgggggcctg gggttttaat ctgttggttt aatacttaac tcatgtatta 840

ttttattttg attcaagtat taaaacccac caaaagtaaa gtgagtggtg cctatgaaat 900

ataataaaac ccatttctga aaatgttcct gtgcattcta tagttctctt ttcctaattg 960

ttgtcagcaa atcactaccc ttgaccttta ttgtacacag ctttgcagca ccctatattg 1020

atataaaaac tcaatataac aaaacaatat aacttcagtc tgatgcttaa caaagaataa 1080

attattctaa gaattcttgt ccataaacta agtatggttt tctatgtcat gatttttttt 1140

tttttttttt ttttttggaa ggggataggg tggaaagaca gagaaaagaa aagggagaaa 1200

aagagtattc caaaggatca agtgctttga gttaactctg ccagtctggg ttaactctgc 1260

cagtctcggt ctgggcaaaa cacagtgaaa agactactat ttccaactag 1310

<210> 25
<211> 1310
<212> DNA
<213> homo sapiens

<400> 25

```
ttatctcctg tcattctcac aataacctta tgaggtaggt attatactta catacatttt      60
gtgggtgtag aaaacttgag ctcagagagg tgaaatgatg tatccaaggt tgcctaacta     120
gctaatggaa gaactaggat ttaagctcat gtttaacctc aaagcctatg tgcataacca     180
caacactgaa aacacagctt gggagaattg agaagaggga gcaaggatgc tgactctagg     240
gtctggtact ttcctcaagt cagggaggca ggatgatgac caaatttcc cctctctgct     300
gcatagagga acccactgaa gcttgggacc cctagaaatg ccatagctac aggactcagg     360
cctgatgagc ttccagctga gcactgggtg tcactcgggc tttacctgct tatcttcagg     420
atctctcttc tggttaggaa ccctatgcct attttgctca cttccctatt ctggtgcttt     480
ggttggtcct ttgagcaaga tccccgggc taagggtgtg acttcatcat tcctttctct     540
ctctttccct ctagggtcac atctcccagg aagatctcac ttccccacag aagctcttgg     600
cctggcctcc tgcagtgcca cgctccgtgt atttgacaag ctgagttgga cactccatgt     660
ggtagagtgt cagtttgtca aataccccaa gtgcggcaca tgcttaccag ctctaggcca     720
gggcagatgg gatatgacga atggactgcc agctggatac aaggatgctc accaagcacc     780
aagttctcac aagttatttt atgtgacttt gcaggaactg aggcattata tctgaggaca     840
ccaggggaaa agtgtggcat ctcagggaaa tacagccctg ggctgtgtct acacacacca     900
tgagagtgct gatggggcg caatagtctt gaaaatgtat aaagtgtcca ggaatggaag     960
tgctctttga ttcattatta ttttcttcct tcatattccc ctcccagagt ctcctatcta    1020
ggacatcagc attctcacac aagcctaatg gcttatctga gtaagcaggg cttagaaatt    1080
cactttcttg atactcagtc ttgccttcta aacactcctt gatcttgcct acctctcccc    1140
ttttccacat gtcttttcct gtaggaacac tttctccatt tattcctgcc tatccaattc    1200
ttccctatat ttcctggacc agctaaagtc cagtgtttcc agagactttt gaaagtcaac    1260
ttacactttt tccttcttca ttcacaaagc tcttcttccc tgggccctgg                1310


<210>  26
<211>  1296
<212>  DNA
<213>  homo sapiens

<400>  26
ttatgagagc tgggctttga aggatttggc tcagaagtca tgagcgatgg gaaggacatg      60
cctggtggag gggacagagt gaggaaaggc tgggagggag caaatgagat gcatcctagg     120
gaagagcccc agaatggtga cgagccccag ctgtggagtc agctggccct gggtgaccct     180
tgaactgatt aggaattccc gaagagaatt cacagctcca ctactgacta gtcgtgttgc     240
tttatctaca gagcctcagt ttctttgtct gtccagtgtg ggtggtgaca atctgggggg     300
tccttgtgag agtctaatgt ggtagcatgc atgtgcctgg cacaatgttg gcactccggg     360
```

```
ttcgcagccg ctgttagtta atgccagcac tcaggcggcc agaggtggat gtaagcccta     420
catccaggac cttgaaggcc taggaggagc catggcagga gccacgggca cctaccagca     480
tccctggggg tgggcagggc ttggtgccgt gctagcatct aacccagccg cgagcttcct     540
tctgcaggtc ctggagccct ggcgtggggc gggcctcctg ccggcgagcg cctgcgccct     600
tccctggcgt gagggtatgt gcctttggac tacatcgtgg aagccagcac catgcagtcc     660
atgggcatat acacttgcct caaggcctat gtcatcgagg agccaccgga gctgccactg     720
ccaccaggga ggaagaggag gagccgggat gtgggatggc agtggtgggt gggctgcggc     780
aggttgggcc agccacacct cactgcttga ccgctctgac cccctttctt ctctttccta     840
gggctacatt gggctcccag ggctcttcgg cctgccaggg tctgatggag aacgagtaag     900
tttgcttctt tggttattca ccatccacag ccacccctgc ccaaacagag cagaacctgc     960
caggcaccag cctggggaag tcaaggaatt cctcctggag gaggtgcact tgagtcgagt    1020
aggagtctga taaggggaca agtcaaggca gatgcattcc aggcataggg aacagcatgt    1080
gcgaagtcag ggatttacgg taacttggcg agtttaacac tcaacactgc caaagtgctg    1140
cgtgagaggg tgagagtggt gggagttgag gctggagagg accctggggc aatgtcttag    1200
gtgcccagga ctaaggttag aaaaggaact ctgttctgag ggtatgcgga gcctcttgag    1260
gacttgcaac caatgaacac ccggcccact ttataa                              1296
```

```
<210>  27
<211>  1272
<212>  DNA
<213>  homo sapiens

<400>  27
aggcgtcccc tctgccatcc gagcgcttgc actgcgcttc cgggaggagg gttgcggccc      60
gtagtcagta gttggggggt gggaacggct tcatacagga gttgatgcac agttatccag     120
ctcctatatg atgcctttct tcatcccctt caaccacgcg cagcccccgg accctcctct     180
gcacccttgg ctgcacgggg acgcgtcctc aagtccgctg ctgtcctgcc agcctcttcc     240
cggcatgctc cttgggctct tctctctctc ttctgcagct ctctgacttc ctggagggga     300
gggcctgggg ctgggccatc tctgaggccc ctccttggca gggaactatg cccctggtgg     360
gacaccccct gggctgactc cacagccaag ggcagatagg ccggaggggg agttgggtag     420
ccctggcgct tctcccactg aggggctggg gctgtgctcc cacacagagc cctccaaagg     480
gacccggccc acaggatgga tggacagacg ttggagagat ggtgcccctt tgtgcccagg     540
gtggaggggg tgggctttgg gcggaccagg gctggccctt tctgtgcccc aagaggaggg     600
tctcctcgag ggtctctgcc ctctacccag gactctttca tgaccaggag gctgaggccc     660
ctcacaggcg gcttcttact ctctccttaa acctgttctg gagacatttc ccctctcccc     720
```

```
aaggattccc aactctgggt ccccagaagc tgtcagctgg gctcccttct tcccctgtgc     780
gtaccctgct caggctgggt acctgagggt gcccaatggc aacatgagca gggggcacgg     840
ccgtggcccc tggttcttgg acctctgtct tctgcctctc tgactaactc aggtcctgtg     900
catggcagag gtgacccgca ccaagccctg tgatattggc atcattaacc ccattaacaa     960
gccacgacag ccagcccagg catgagcatc ggggcagcag ggcagccagt gtggggagag    1020
aggccttgca gtagctgagg ccattagaga ggacaagagg gtgcaggagg gagcatcctc    1080
acctctgccc ttcccactta catcccatct cccccttcctc acccctcctt ccccatgcac   1140
cccttcttcc tgtaggaggg agcaccctca cctctgccct tcccactcac ctcccatctt    1200
cccttttctca ccccccctcc ccaatgcacc ccctcccttc ctgcaggagg gagcaccctc   1260
tcccctgccc tt                                                        1272
```

## Description

The present invention relates to nucleic acid molecules comprising at least ten contiguous bases having a sequence as shown in the sequence of microRNA(miR)-223 (SEQ ID NO: 16) for treating fibrosis. Also methods for treating fibrosis are provided.

MicroRNAs are a broad class of small non-coding RNAs that control diverse biological processes including major signaling pathways by regulating the expression of complementary target mRNAs (Ambros, 2004). Dysregulation of microRNAs in various disease entities is caused by alterations in the genome (Mi et al., 2007), differential expression or viral infections in some cases changing microRNA function into tumor suppressors or oncogenes. MicroRNAs were recently implicated in the regulation of diverse cardiac functions in a series of genetic studies (Care et al., 2007; Yang et al., 2007). Although these studies help to delineate the roles of microRNA in heart physiology, growth and morphogenesis, detailed molecular mechanisms for microRNAs in disease pathways in vivo are poorly understood. Single-stranded oligonucleotide microRNA antagonists have been shown to silence endogenous microRNAs in vitro and in vivo with resulting effects on target mRNA and protein levels and metabolism (Kruetzfeldt et al., 2005; Esau et al., 2006). These findings pointed to the application of microRNA antagonists for in vivo validation of microRNA function and, perhaps more importantly, as a novel therapeutic modality.

Recently, some studies have suggested that single microRNAs may play an important role in cardiac fibrosis. Cardiac fibrosis is a major event occuring during the development of heart failure. Cardiac fibrosis is known as an accumulation of extracellular matrix, which is secreted into the myocardium by cardiac fibroblasts (CFs). This process is associated with strong changes to the transcriptional activity in CFs.

Conventional therapy of fibrosis often comprises the destruction of myofibroblasts by natural killer cells. Also the use of inhibitors of the signal transduction by signalling molecules such as TGFbeta, CTGF or p38-MAPK is discussed in the art. It is also tried to counteract the activity of compounds known to activate TGFbeta, like Angiotensin or Aldosteron. However, these approaches may lead to severe side effects or lack efficiency. Accordingly, improved fibrosis therapies are highly desirable.

Thus, the technical problem underlying the present invention is the provision of means and methods for the medical intervention in fibrosis.

The technical problem is solved by provision of the embodiments characterized in the claims.

Accordingly, the present invention relates to nucleic acid molecules comprising at least ten contiguous bases having a sequence as shown in the sequence of miR-223 (SEQ ID NO: 16) for treating fibrosis.

Understanding the expression pattern and roles of miRNAs in cardiac fibroblasts (CF) may lead to the development of therapeutics to interfere with the progression of fibrosis, e.g. cardiac fibrosis, and thus be useful in the treatment of heart failure. To this end, a microarray-based approach was developed to determine the miRNA expression pattern in CFs from a murine heart failure model and healthy control individuals. A set of microRNAs was sucessfully identified which are selectively expressed in CFs as opposed to other cardiac cell types and which are dysregulated during cardiac failure.

Moreover, a functional *in vitro* screening strategy was developed in order to find out in which general cellular functions these microRNAs are involved. As demonstrated in the appended examples, it was surprisingly found that miR-223 has a potent antiproliferative effect in neonatal rat fibroblasts.

As shown herein, miR-223 has been identified as a particularly potent anti-proliferative agent; see Fig. 1. Surprisingly, only one of the miRNAs tested, namely miR-223 showed a pronounced antiproliferative activity, while the other miRNAs enhanced proliferation. In contrast, the proliferation of fibroblasts was significantly diminished by miR-223 by about 50 % compared to the control; see Fig. 1 and 2. Since fibrosis is associated with proliferation of fibroblasts, miR-223 can be successfully used to prevent the development of fibrosis and/or to treat fibrosis. Moreover, it has been found herein that the expression of miR-223 is decreased in failing cardiac fibroblasts; see Fig. 3. In accordance with the above, it is believed that the anti-proliferative activity of miR-223 is reduced in failing cardiac fibroblasts and that the nucleic acid molecules comprising at least ten contiguous bases having a sequence of bases as shown in the sequence of miR-223 (SEQ ID NO: 16) can, accordingly, be successfully used in the intervention of fibrosis, for example cardiac fibrosis.

As mentioned above, the nucleic acid molecules defined herein, are particularly useful in the treatment of fibrosis. The term "fibrosis" is well known in the art. As used herein the term "fibrosis" is to be understood as the formation or development of excess fibrous connective tissue in an organ or tissue as a reparative or reactive process, as opposed to a formation of fibrous tissue as a normal constituent of an organ or tissue.

The invention particularly concerns cardiac fibrosis, and in particular cardiac diseases that involve fibrosis, such as heart failure, cardiac hypertrophy, heart-related storage disease and the like. Further fibrotic diseases to be treated herein are described below in more detail.

The term "microRNA" or "miRNA" is well known in the art and used accordingly herein; see, for example, Bartel, Cell, 2004, 116, 281-297 for a review on microRNA molecules). miRNAs are derived from genomic loci and are produced from specific microRNA genes. Transcription of these microRNA genes usually by RNA polymerase II gives rise to so called "pri-miRNA". In mammals this pri-miRNA is cleaved by the RNAse III Drosha and a cofactor DGCR8/Pasha. After Exportin-5 mediated transport from the nucleus in the cytoplasm the then "pre-miRNA" (precursor miRNAs) is further processed by Dicer in mature miRNAs.

The pre-miRNAs form local hairpin structures and mature microRNAs are processed from these precursor transcripts, by cleavage of the hairpin structures by Dicer, generating thereby one microRNA duplex. This microRNA duplex typically consists of two strands, one being the mature miRNA (miR), the other being a complementary strand (miR*), wherein the latter is usually degraded. Generally, one of the two strands of a microRNA duplex (usually the mature miR) is packaged in a microRNA ribonucleoprotein complex (microRNP). It is generally thought that mature mammalian microRNAs bind to mature mRNA and inhibit translation of the respective mRNA.

The miR-223 used in context of the present invention is well known in the art. In accordance with the above nomenclature, the term "miR-223" denotes a mature miRNA (miR) which is derived from the precursor (hairpin/ stem loop) sequence (pre-miRNA). A sequence of the pre-miRNA of miRNA-223 is given herein in SEQ ID NO: 8 (also publicly available in the miRbase database under Accession No. MI0000300. The mature miR sequence of miR-223 is shown herein in SEQ ID No. 16 (Accession No. MIMAT0000280 of miRbase).

As demonstrated in the appended examples, the nucleic acid molecules comprising a pre-miRNA (as shown in SEQ ID NO: 8) of miR-223 (as shown in SEQ ID NO: 16) can successfully be used in the treatment of fibrosis. For example, the nucleic acid molecule comprising pre-miRNA may be delivered by various modes of administration (described herein below in more detail) to the affected/diseases cell, tissue and/or organ, where the pre-miRNA molecule is then further processed by cellular mechanisms into mature miRNA (i.e. nucleic acid molecule comprising or consisting of miR-223 (SEQ ID NO: 8). The miR-223 (SEQ ID NO: 16) sequence portion corresponds to nucleotides 68 -89 of SEQ ID NO: 8. The mature miRNA which is then thought to exert the anti-proliferative action. However, it is also envisaged herein that a nucleic acid molecule comprising/consisting of the mature miRNA (SEQ ID NO: 16), optionally with modifications, or the miR/miR* duplex (also optionally modified) is used in accordance with the present invention.

The nucleic acid molecule to be used herein is typically an RNA molecule. The nucleic acid molecule may comprise at least ten contiguous bases having a sequence as shown in SEQ ID NO: 16 (mature miR-223 sequence). Preferably, the nucleic acid molecule comprises at least thirteen, more preferably at least fifteen, and even more preferably at least twenty or twenty-one contiguous bases having a sequence of bases as shown in the sequence of miR-223 (SEQ ID NO: 16). Most preferably, the nucleic acid molecule comprises twenty-two contiguous bases having a sequence of bases as shown in the sequence of miR-223 (SEQ ID NO: 16), i.e. comprises the bases as shown in the miR-223 sequence (SEQ ID NO: 16). Also preferred herein is a nucleic acid molecule which comprises at least the "seed sequence" of miR-223 as shown in SEQ ID NO: 19. The miR-223 seed sequence (SEQ ID NO: 19) corresponds to nucleotides 69 to 74 of SEQ ID NO: 8 (pre-miR-223) and to nucleotides 2 to 7 of SEQ ID NO: 16 (miR-223) Accordingly, the nucleic acid molecule may also comprise at least 7 contiguous bases, provided that the miR-223 seed sequence is contained in the nucleic acid molecule. It is preferred herein that the nucleic acid molecule as defined herein comprises the miR-223 seed sequence as shown in SEQ ID NO: 19, preferably the unaltered (no additions, deletions and/or mismatches) contiguous bases as shown in SEQ ID NO: 19.

Also the following sequence can be considered as seed sequence of miR-223: GUCAGU This sequence corresponds to nucleotides 1 to 6 of SEQ ID NO: 19. All explanations given herein in respect of the seed sequence shown in SEQ ID NO: 19 apply, mutatis mutandis, also to the seed sequence GUCAGU.

In particular it is preferred herein that the herein defined and disclosed nucleic acid molecules which are to be used in accordance with the present invention, contain the seed sequences shown in SEQ ID NO: 19 and/or given in the sequence of GUCAGU. It is particularly preferred that the nucleic acid molecules to be used herein, and in particular the nucleic molecules containing the above defined seed sequence(s), contain nucleotides 1 and 8 of the sequence as shown in SEQ ID NO: 16 (mature miR-223).

The term "base" as used herein refers to any one of the nucleotide bases normally found in naturally occurring DNA or RNA. The bases can be purines or pyrimidines. Examples of purine bases include adenine (A) and guanine (G). Examples of pyrimidine bases include thymine (T), cytosine (C) and uracil (U). The adenine can be replaced with 2,6-diaminopurine.

Sequences of (unmodified) nucleic acid molecules (RNA molecules) disclosed and used herein may have uracil bases. Though less preferred, also the use of (unmodified) DNA molecules is envisaged. The sequence of bases of the (unmodified) DNA molecule is the same as of the unmodified RNA molecule, except that in the unmodified DNA molecule, the uracil bases are replaced with thymine bases. It is well known in the art that each base in the sequence can form a Watson-Crick base pair with a complementary base. Watson-Crick base pairs as used herein refer to the hydrogen bonding interaction between, for example, the following bases: adenine and thymine (A-T); adenine and uracil (A-U); and cytosine and guanine (C-G).

In a further embodiment the present invention relates to a nucleic acid molecule for treating fibrosis, wherein up to 10 % of the contiguous bases are non-complementary.

The term "non-complementary" as used herein refers to additions, deletions, mismatches or combinations thereof. Additions refer to the insertion in the contiguous sequence of any base described above. Deletions refer to the removal of any moiety present in the contiguous sequence. Mismatches refer to the substitution of one of the bases in the contiguous sequence with a different base. The additions, deletions or mismatches can occur anywhere in the contiguous sequence, for example, at either end of the contiguous sequence or within the contiguous sequence of the molecule. Typically, the additions, deletions or mismatches occur at the end of the contiguous if the contiguous sequence is relatively short, such as, for example, from about ten to about fifteen bases in length. If the contiguous sequence is relatively long, such as, for example, a minimum of sixteen contiguous bases in length, the additions, deletions, or mismatches may occur anywhere in the contiguous sequence.

For example, none or one base in the contiguous bases may be added, deleted or replaced by a mismatch when the number of contiguous bases is ten to nineteen; and a maximum of one or two bases may be added, deleted, or replaced by a mismatch, when the number of contiguous bases is twenty to twenty-two. Further, no more than fifty percent, and preferably no more than thirty percent, of the contiguous bases in a nucleic acid molecule contain deoxyribonucleotide backbone units. Preferably, the contiguous bases contains essentially ribonucleotide backbone units. The same hold true for the nucleic acid molecule as a whole. Though, the nucleic acid molecule may be a chimeric molecule comprising deoxyribonucleotide backbone units and ribonucleotide backbone units, it is preferred that the nucleic acid molecule is an RNA, molecule.

As an overall principle, it is preferred herein that the nucleic acid molecule to be used herein has an activity that is substantially the same as the mature miR-223 (SEQ ID NO: 16). Means and methods for measuring this activity are described herein and are also demonstrated in the appended examples. For example, the activity of the mature miR-223 is reflected in the anti-proliferative effect on fibroblasts; see Fig. 1 and 2. Preferably, the nucleic acid molecule used herein differs less than 10 % from the activity of the mature miR-223.

The nucleic acid molecule used in accordance with the present invention may, in addition to the at least ten contiguous bases (or in addition to the at least seven contiguous bases if these seven bases comprise the miR-223 seed sequence (SEQ ID NO: 19) as defined above, comprise one or more additional bases. Accordingly, the present invention relates to a nucleic acid molecule as defined and disclosed herein above, which further comprises at least one base at the 5' end and/or at least one base at the 3' end.

Though there is no upper limit to the number of bases to be added at the 5' end and/or at the 3' end, typically, no more than about 500 nucleotides, and preferably no more than about 300 nucleotides are added to either end of the at least ten contiguous bases. The overall length of the nucleotide molecule to be used herein does usually not exceed several 100 bases, e.g. 500 bases, 400 bases or 300 bases, more preferably 200 or 100 bases, even more preferably 50 bases. Typically the overall length of the nucleotide moleculelies in the range of 15 to 30 bases, preferably in the range of 18 to 25 bases. Generally, any nucleotide can be added, such as the bases described above. Thus, for example, the additional nucleotides may be any one or more of A, G, C, T, or U.

As mentioned above, the mature miR-223 molecule is part of the miR-223 precursor molecule (stem loop precursor, hairpin precursor) or of a fragment thereof. Accordingly, nucleic acid molecules to be used in accordance with the present invention may not only comprise the mature miR-223 molecule (SEQ ID NO: 16), they may also comprise the miR-223 precursor molecule having a sequence as shown in SEQ ID NO: 8 or a fragment thereof. In preferred embodiments, the nucleic acid molecule disclosed herein consists of a molecule having a sequence as shown in SEQ ID NO: 16 (mature miR-223) or consists of the miR-223 precursor molecule having a sequence as shown in SEQ ID NO: 8. As mentioned above, also the use of a duplex comprising the nucleic acid molecule as defined herein, in particular a molecule having a sequence of the mature miR-223 (SEQ ID NO: 16), and a complementary strand thereto is envisaged. In this particular embodiment, the nucleic acid molecule to be used herein is single-stranded and may, thus, form a duplex with a complementary strand (also single-stranded).

It is to be understood that the miR-223 precursor molecules and fragments thereof usually form hairpins, though the nucleic acid molecule as defined and used herein is single-stranded. This is to be distinguished from the situation where a duplex of the single-stranded nucleic acid molecule disclosed herein is formed with a separate single-stranded molecule having an (at least partially) complementary sequence to the sequence of the nucleic acid molecule. In other words, the duplex to be used herein, comprises or consists of two individual nucleic acid molecules having (at least partially) complementary sequences. Usually, such a complex is formed after transfection of a cell with the miR-223 precursor molecule (or a (recombinant) vector carrying it), whereby the duplex is then excised from the precursor molecule. However, the duplex may also be produced in vitro and then introduced into the cell.

The fragment of the hairpin precursor sequence may, for example, contain at least ten, preferably at least fifteen, more preferably at least twenty bases of the miR-223 precursor molecule. It is preferred herein that the sequence of the mature miR-223 molecule (and also the seed sequence of the mature miR-223) is comprised in the sequence of the fragment of the hairpin precursor and/or the hairpin precursor itself.

The nucleic acid molecule as defined and described herein can be inserted into a vector, such as, for example, a recombinant vector. Typically, to construct a recombinant vector containing a nucleic acid molecule, the hairpin miR-223 precursor molecule or a fragment thereof (which, preferably, contains the a portion having the sequence of the mature miR-223 or the miR-223 seed sequence) is incorporated into the vector; see for example, Chen et al. Science 2004, 3033:83-86. The recombinant vector may be any recombinant vector, such as a plasmid, a cosmid or a phage. Recombinant vectors generally have an origin of replication. The vector may be, for example, a viral vector, such as an adenovirus vector or an adeno-associated virus (AAV) vector; see for example Ledley 1996, Pharmaceutical Research 13:1595-1614 and Verma et al. Nature 1997, 387:239-242. The vector may further include a selectable marker. Suitable selectable markers include a drug resistance marker, such as tetracycline or gentamycin, or a detectable gene marker, such as beta-galactosidase or luciferase.

The nucleic acid molecule to be used herein is an isolated molecule. In context of the present invention, the term "isolated" means that the molecule is essentially free of other nucleic acids. Essentially free from other nucleic acids means that the molecule is at least about 90%, preferably at least about 95% and, more preferably at least about 98% free of other nucleic acids. Preferably, the molecule is essentially pure, which means that the molecules are free not only of other nucleic acids, but also of other materials used in the synthesis and isolation of the molecule. Materials used in synthesis include, for example, enzymes. Materials used in isolation include, for example, gels, such as SDS-PAGE. The molecule is at least about 90% free, preferably at least about 95% free and, more preferably at least about 98% free of such materials.

It is well known in the art that the sequence of bases in a microRNA or hairpin precursor is highly conserved. Thus, also the use of nucleic acid molecules having a homologous sequence to the sequence of the herein above described nucleic acid molecules is envisaged in context of the present invention. For example, the sequences of the human, murine and rat miR-223 are highly homologous and may, accordingly, be used herein. However, the sequence can be from the miR-223 of any mammal. Exemplary mammals include pet animals, such as dogs and cats, farm animals, such as cows, horses and sheeps, laboratory animals, such as rats, mice and rabbits, and primates, such as monkeys and humans. Preferably, the mammal is human, rat or mouse.

The term "miR-223" has been defined and described herein above. In the sequence listing as provided herein, the human mature miR-223 nucleic acid sequence is provided as SEQ ID NO: 16. As mentioned above, not only miR-223 (SEQ ID NO: 16) but also homologous sequences, wherein the definitions and explanations given herein above in context of nucleic acid molecules comprising at least ten contiguous bases having a sequence of bases as shown in the sequence of miR-223 (SEQ ID NO: 16) also apply to the homologous sequences. Homologous sequences can be obtained from the corresponding databases, e.g. miRbase, NCBI and the like. In accordance with the above, the use of nucleic acid molecules hybridizing under stringent conditions to the complementary strand of nucleic acid molecules having a sequence as shown in SEQ ID NO: 16 and having the activity of miR-223 (SEQ ID NO: 16) and/or having at least 60 % homology to the sequence as shown in SEQ ID NO: 16 and having the activity of miR-223 (SEQ ID NO: 16) is envisaged herein in the treatment of fibrosis.

The nucleic acid sequence of miR-223 of other species than the herein provided sequence for miR-223 (SEQ ID NO: 16) can, accordingly, be identified by the skilled person using methods known in the art, e.g. by using hybridization assays or by using alignments, either manually or by using computer programs such as those mentioned herein below in connection with the definition of the term "hybridization" and degrees of homology. In one embodiment, the nucleic acid sequence of orthologs of miR-223 is at least 40% homologous to the nucleic acid sequence as shown in SEQ ID NO. 16. More preferably, the nucleic acid sequence of orthologs of miR-223 is at least 45%, 50%, 55%, 60%, 65%, 70%, 75%, 80%, 85%, 90%, 91%, 92%, 93%, 94%, 95%, 96%, 97% or 98% homologous to the nucleic acid sequence as shown in SEQ ID NO. 16, wherein the higher values are preferred. Most preferably, the nucleic acid sequence of orthologs of miR-223 is at least 99% homologous to the nucleic acid sequence as shown in SEQ ID NO. 16.

The term "orthologous" or "orthologous gene" as used herein refers to miR-223 variants in different species that are similar to each other because they originated from a common ancestor.

Hybridization assays for the characterization of orthologs of known nucleic acid sequences are well known in the art; see e.g. Sambrook, Russell "Molecular Cloning, A Laboratory Manual", Cold Spring Harbor Laboratory, N.Y. (2001); Ausubel, "Current Protocols in Molecular Biology", Green Publishing Associates and Wiley Interscience, N.Y. (1989).

The term "hybridization" or "hybridizes" as used herein may relate to hybridizations under stringent or non-stringent conditions. If not further specified, the conditions are preferably non-stringent. Said hybridization conditions may be established according to conventional protocols described, e.g., in Sambrook (2001) loc. cit.; Ausubel (1989) loc. cit., or Higgins and Hames (Eds.) "Nucleic acid hybridization, a practical approach" IRL Press Oxford, Washington DC, (1985). The setting of conditions is well within the skill of the artisan and can be determined according to protocols described in the art. Thus, the detection of only specifically hybridizing sequences will usually require stringent hybridization and washing conditions such as, for example, the highly stringent hybridization conditions of 0.1 x SSC, 0.1% SDS at 65°C or 2 x SSC, 60°C, 0.1 % SDS. Low stringent hybridization conditions for the detection of homologous or not exactly complementary sequences may, for example, be set at 6 x SSC, 1% SDS at 65°C. As is well known, the length of the probe and the composition of the nucleic acid to be determined constitute further parameters of the hybridization conditions.

In accordance with the present invention, the terms "homology" or "percent homology" or "identical" or "percent identity" or "percentage identity" or "sequence identity" in the context of two or more nucleic acid sequences refers to two or more sequences or subsequences that are the same, or that have a specified percentage of nucleotides that are the same (preferably at least 40% identity, more preferably at least 45%, 50%, 55%, 60%, preferably 65%, more preferably 70%, even more preferably 75%, 80%, 85%, 90%, 91%, 92%, 93%, 94%, 95%, 96%, 97% or 98% identity, most preferably at least 99% identity), when compared and aligned for maximum correspondence over a window of comparison, or over a designated region as measured using a sequence comparison algorithm as known in the art, or by manual alignment and visual inspection. Sequences having, for example, 75% to 90% or greater sequence identity may be considered to be substantially identical. Such a definition also applies to the complement of a test sequence. Preferably the described identity exists over a region that is at least about 10 nucleotides in length, more preferably, over a region that is at least about 13 to 15 nucleotides in length and most preferably, over a region that is at least about 20 nucleotides in length. Those having skill in the art will know how to determine percent identity between/among sequences using, for example, algorithms such as those based on CLUSTALW computer program (Thompson Nucl. Acids Res. 2 (1994), 4673-4680) or FASTDB (Brutlag Comp. App. Biosci. 6 (1990), 237-245), as known in the art.

Although the FASTDB algorithm typically does not consider internal non-matching deletions or additions in sequences, i.e., gaps, in its calculation, this can be corrected manually to avoid an overestimation of the % identity. CLUSTALW, however, does take sequence gaps into account in its identity calculations. Also available to those having skill in this art are the BLAST and BLAST 2.0 algorithms (Altschul, (1997) Nucl. Acids Res. 25:3389-3402; Altschul (1993) J. Mol. Evol. 36:290-300; Altschul (1990) J. Mol. Biol. 215:403-410). The BLASTN program for nucleic acid sequences uses as defaults a word length (W) of 11, an expectation (E) of 10, M=5, N=4, and a comparison of both strands. The BLOSUM62 scoring matrix (Henikoff (1989) PNAS 89:10915) uses alignments (B) of 50, expectation (E) of 10, M=5, N=4, and a comparison of both strands.

In order to determine whether an nucleotide residue in a nucleic acid sequence corresponds to a certain position in the nucleotide sequence of e.g. SEQ ID NO: 16, the skilled person can use means and methods well-known in the art, e.g., alignments, either manually or by using computer programs such as those mentioned herein. For example, BLAST 2.0, which stands for Basic Local Alignment Search Tool BLAST (Altschul (1997), loc. cit.; Altschul (1993), loc. cit.; Altschul (1990), loc. cit.), can be used to search for local sequence alignments. BLAST, as discussed above, produces alignments of nucleotide sequences to determine sequence similarity. Because of the local nature of the alignments, BLAST is especially useful in determining exact matches or in identifying similar sequences. The fundamental unit of BLAST algorithm output is the High-scoring Segment Pair (HSP). An HSP consists of two sequence fragments of arbitrary but equal lengths whose alignment is locally maximal and for which the alignment score meets or exceeds a threshold or cut-off score set by the user. The BLAST approach is to look for HSP between a query sequence and a database sequence, to evaluate the statistical significance of any matches found, and to report only those matches which satisfy the user-selected threshold of significance. The parameter E establishes the statistically significant threshold for reporting database sequence matches. E is interpreted as the upper bound of the expected frequency of chance occurrence of an HSP (or set of HSPs) within the context of the entire database search. Any database sequence whose match satisfies E is reported in the program output.

Analogous computer techniques using BLAST (Altschul (1997), loc. cit.; Altschul (1993), loc. cit.; Altschul (1990), loc. cit.) are used to search for identical or related molecules in nucleotide databases such as GenBank or EMBL. This analysis is much faster than multiple membrane-based hybridizations. In addition, the sensitivity of the computer search can be modified to determine whether any particular match is categorized as exact or similar. The basis of the search is the product score which is defined as:

$$\frac{\%\ \text{sequence identity}\ \times\ \%\ \text{maximum BLAST score}}{100}$$

and it takes into account both the degree of similarity between two sequences and the length of the sequence match. For example, with a product score of 40, the match will be exact within a 1-2% error; and at 70, the match will be exact. Similar molecules are usually identified by selecting those which show product scores between 15 and 40, although lower scores may identify related molecules. Another example for a program capable of generating sequence alignments is the CLUSTALW computer program (Thompson (1994) Nucl. Acids Res. 2:4673-4680) or FASTDB (Brutlag (1990) Comp. App. Biosci. 6:237-245), as known in the art.

The following relates to modified nucleic acid molecules to be used in accordance of the present invention.

The explanations and definitions given herein above in respect of the nucleic acid molecule to be used in accordance of the present invention (e.g. the explanations regarding the sequence, the number of contiguous bases, the non-complementary bases, the bases that may be added at the 5' and/or the 3' end etc.) apply, mutatis, mutandis, also to the modified nucleic acid molecules described herein below, if not specific explanation on modified nucleic acid molecules are given below. For example, the bases that may be added, deleted or replaced by mismatches in the contiguous base sequence may be modified bases. Similarly, bases to be added to the at least ten contiguous bases as defined above, may be modified bases. A person skilled in the art is readily in the position to adapt the teaching given herein below regarding modified bases/modified nucleotides to the nucleic acid molecule as defined herein above.

The present invention relates in a further embodiment to a modified nucleic acid molecule as defined and described herein above. The modified nucleic acid molecule can have the same sequence as any of the nucleic acid molecules described herein above, e.g. a nucleic acid molecule comprising at least ten contiguous bases having a sequence of bases as shown in SEQ ID NO: 16 (mature miR-223), such as the mature miR-223 itself (shown in SEQ ID NO: 16) or the miR-223 precursor molecule (shown in SEQ ID NO: 8). However, the modified nucleic acid molecule comprises at least one modified moiety (in other words, at least one moiety of the modified nucleic acid molecule is not an unmodified deoxyribonucleotide moiety or ribonucleotide moiety).

The modified nucleic acid molecule comprises a minimum number of ten modified moieties, preferably a minimum of thirteen, more preferably a minimum of fifteen, even more preferably a minimum of eighteen, and even more preferably a minimum of twenty-one modified moieties. Most preferably, the modified nucleic acid molecule comprises twenty-two modified moieties.

The modified nucleic acid molecules comprise a maximum number of fifty modified moieties, preferably a maximum of forty, more preferably a maximum of thirty, even more preferably a maximum of twenty-five, and most preferably a maximum of twenty-three modified moieties. A suitable range of minimum and maximum numbers of modified moieties may be obtained by combining any of the above minima with any of the above maxima.

Moreover, any number of additional moieties, up to a maximum of forty moieties, having any base sequence can be added to the moieties comprising the contiguous base sequence, as long as the total number of moieties in the molecule does not exceed fifty. The additional moieties can be added to the 5' end, the 3' end, or to both ends of the contiguous sequence. The additional moieties can include a sequence of bases at the 5' end and/or a sequence of bases at the 3' end present in the hairpin precursor from which the mature miR-223 molecule is derived or any fragment thereof. The additional moieties in the molecule, if any, can be any modified or unmodified moiety described herein.

Each modified moiety comprises a base bonded to a backbone unit. The backbone unit may be any molecular unit that is able to stably bind to a base and to form an oligomeric chain. As used herein the backbone units of a modified moiety do not include the backbone units commonly found in naturally occurring DNA or RNA molecules.

Such modified nucleic acid molecules have increased nuclease resistance. Therefore, the nuclease resistance of the molecule is increased compared to a molecule containing only unmodified ribonucleotide moieties, unmodified deoxyribonucleotide moieties or both. Such modified moieties are well known in the art, and were reviewed, for example, by Kurreck, Eur. J. Biochem. 270, 1628-1644 (2003).

The nuclease resistance can be a resistance to an exonuclease, an endonuclease, or both. The exonuclease can be a 3' to 5' exonuclease or a 5' to3' exonuclease. Examples of 3' to 5' human exonuclease include PNPT1, Werner syndrome helicase, RRP40, RRP41, RRP42, RRP45, and RRP46. Examples of 5' to 3' exonuclease include XRN2, and FEN1. Examples of endonucleases include Dicer, Drosha, RNase4, Ribonuclease P, Ribonuclease HI, DHP1, ERCC-1 and OGG1. Examples of nucleases which function as both an exonuclease and an endonuclease include APE1 and EXO1.

A modified moiety can occur at any position in the nucleic acid molecule. For example, to protect nucleic acid molecules against 3' to 5' exonucleases, the molecules can have at least one modified moiety at the 3' end of the molecule and preferably at least two modified moieties at the 3' end. If it is desirable to protect the molecule against 5' to 3' exonuclease, the nucleic acid molecules can have at least one modified moiety and preferably at least two modified moieties at the 5' end of the molecule. The nucleic acid molecules can also have at least one and preferably at least two modified moieties between the 5' and 3' end of the molecule to increase resistance of the molecule to endonucleases. Preferably, at least about 10%, more preferably at least about 25%, even more preferably at least about 50%, and further more preferably at least about 75%, and most preferably at least about 95% of the moieties are modified. In one embodiment, all of the moieties are modified (e.g., nuclease resistant).

In one example of a modified nucleic acid molecule (in particular a DNA molecule), the molecule comprises at least one modified deoxyribonucleotide moiety. Suitable modified deoxyribonucleotide moieties are known in the art. Such modified deoxyribonucleotide moieties comprise, for example, phosphorothioate deoxyribose groups as the backbone unit. A modified nucleic acid molecule comprising phosphorothioate deoxyribonucleotide moieties is generally referred to as phosphorothioate (PS) DNA; see, for example, Eckstein, Antisense Nucleic Acids Drug Dev. 10,117-121 (2000).

Another suitable example of a modified deoxyribonucleotide moiety is an N'3-N'5 phosphoroamidate deoxyribonucleotide moiety, which comprises an N'3-N'5 phosphoroamidate deoxyribose group as the backbone unit. An nucleid acid molecule comprising phosphoroamidate deoxyribonucleotide moieties is generally referred to as phosphoroamidate (NP) DNA; see, for example, Gryaznov et al., J. Am. Chem. Soc. 116, 3143-3144 (1994).

In another example of a modified nucleic acid molecule (in particular an RNA molecule), the molecule comprises at least one modified ribonucleotide moiety. A suitable example of a modified ribonucleotide moiety is a ribonucleotide moiety that is substituted at the 2' position. The substituents at the 2' position may, for example, be a $C_1$ to $C_4$ alkyl group. The $C_1$ to $C_4$ alkyl group may be saturated or unsaturated, and unbranched or branched. Some examples of $C_1$ to $C_4$ alkyl groups include ethyl, isopropyl, and allyl. The preferred $C_1$ to $C_4$ alkyl group is methyl. An oligoribonucleotide molecule comprising ribonucleotide moieties substituted at the 2' position with a $C_1$ to $C_4$ alkyl group is generally referred to as a 2'-O--( $C_1$ to $C_4$ alkyl) RNA, e.g., 2'-O-methyl RNA (OMe RNA).

Another suitable example of a substituent at the 2' position of a modified ribonucleotide moiety is a $C_1$ to $C_4$ alkoxy- $C_1$ to $C_4$ alkyl group. The $C_1$ to $C_4$ alkoxy (alkyloxy) and $C_1$ to $C_4$ alkyl group may comprise any of the alkyl groups described above. The preferred $C_1$ to $C_4$ alkoxy- $C_1$ to $C_4$ alkyl group is methoxyethyl. An nucleic acid molecule comprising more than one ribonucleotide moiety that is substituted at the 2' position with a $C_1$ to $C_4$ alkoxy-C.sub.1 to C.sub.4 alkyl group is referred to as a 2'-O--( $C_1$ to $C_4$ alkoxy- $C_1$ to $C_4$ alkyl) RNA, e.g., 2'-O-methoxyethyl RNA (MOE RNA).

Yet another suitable example of a modified ribonucleotide moiety is a ribonucleotide that has a methylene bridge between the 2'-oxygen atom and the 4'-carbon atom. An ribonucleotide molecule comprising ribonucleotide moieties that has a methylene bridge between the 2'-oxygen atom and the 4'-carbon atom is generally referred to as locked nucleic acid (LNA); see, for example, Kurreck et al., Nucleic Acids Res. 30, 1911-1918 (2002); Elayadi et al., Curr. Opinion Invest. Drugs 2, 558-561 (2001); .Orum et al., Curr. Opinion Mol. Ther. 3, 239-243 (2001); Koshkin et al., Tetrahedron 54, 3607-3630 (1998); Obika et al., Tetrahedron Lett.39, 5401-5404 (1998). Locked nucleic acids are commercially available for example from Proligo (Paris, France and Boulder, Colo., USA).

Another suitable example of a modified ribonucleotide moiety is a ribonucleotide that is substituted at the 2' position with fluoro group. Such 2'-fluororibonucleotide moieties are known in the art. Molecules comprising 2'-fluororibonucleotide moieties are generally referred to herein as 2'-fluororibonucleic acids (FANA); see Damha et al., J. Am. Chem. Soc. 120, 12976-12977 (1998).

Another example of a modified nucleic acid molecule relates to a molecule that comprises at least one modified moiety comprising a base bonded to an amino acid residue as the backbone unit. Modified moieties that have at least one base bonded to an amino acid residue will be referred to herein as peptide nucleic acid (PNA) moieties. Such moieties are nuclease resistance, and are known in the art. Molecules having PNA moieties are generally referred to as peptide nucleic acids; see Nielson, Methods Enzymol. 313, 156-164 (1999); Elayadi, et al, id.; Braasch et al., Biochemistry 41, 4503-4509 (2002), Nielsen et al., Science 254, 1497-1500 (1991).

The amino acids can be any amino acid, including natural or non-natural amino acids. Naturally occurring amino acids include, for example, the twenty most common amino acids normally found in proteins, i.e., alanine (Ala), arginine (Arg), asparagine (Asn), aspartic acid (Asp), cysteine (Cys), glutamine (Glu), glutamic acid (Glu), glycine (Gly), histidine (His), isoleucine (Ileu), leucine (Leu), lysine (Lys), methionine (Met), phenylalanine (Phe), proline (Pro), serine (Ser), threonine (Thr), tryptophan, (Trp), tyrosine (Tyr), and valine (Val).

The non-natural amino acids may, for example, comprise alkyl, aryl, or alkylaryl groups. Some examples of alkyl amino acids include alpha.-aminobutyric acid, beta.-aminobutyric acid, gamma.-aminobutyric acid, delta.-aminovaleric acid, and epsilon.-aminocaproic acid. Some examples of aryl amino acids include ortho-, meta, and para-aminobenzoic acid. Some examples of alkylaryl amino acids include ortho-, meta-, and para-aminophenylacetic acid, and gamma.-phenyl-beta-aminobutyric acid.

Non-naturally occurring amino acids also include derivatives of naturally occurring amino acids. The derivative of a naturally occurring amino acid may, for example, include the addition or one or more chemical groups to the naturally occurring amino acid. For example, one or more chemical groups can be added to one or more of the 2', 3', 4', 5', or 6' position of the aromatic ring of a phenylalanine or tyrosine residue, or the 4', 5', 6', or 7' position of the benzo ring of a tryptophan residue. The group can be any chemical group that can be added to an aromatic ring. Some examples of such groups include hydroxyl, C.sub.1-C.sub.4 alkoxy, amino, methylamino, dimethylamino, nitro, halo (i.e., fluoro, chloro, bromo, or iodo), or branched or unbranched C.sub.1-C.sub.4 alkyl, such as methyl, ethyl, n-propyl, isopropyl, butyl, isobutyl, or t-butyl. Other examples of non-naturally occurring amino acids which are derivatives of naturally occurring amino acids include norvaline (Nva), norleucine (Nle), and hydroxyproline (Hyp).

The amino acids can be identical or different from one another. Bases are attached to the amino acid unit by molecular linkages. Examples of linkages are methylene carbonyl, ethylene carbonyl and ethyl linkages. (Nielsen et al., Peptide Nucleic Acids-Protocols and Applications, Horizon Scientific Press, pages 1-19; Nielsen et al., Science 254: 1497-1500.) One example of an amino acid residue of a PNA moiety is N-(2-aminoethyl)-glycine.

Further examples of PNA moieties include cyclohexyl PNA, retro-inverso PNA, phosphone PNA, propionyl PNA and aminoproline PNA moieties. For a description of these PNA moieties, see FIG. 5 of Nielsen et al., Peptide Nucleic Acids-Protocols and Applications, Horizon Scientific Press, pages 1-19.

PNA can be chemically synthesized by methods known in the art, e.g. by modified Fmoc or tBoc peptide synthesis protocols. The PNA has many desirable properties, including high melting temperatures (Tm), high base-pairing specificity with nucleic acid and an uncharged molecular backbone. Additionally, the PNA does not confer RNase H sensitivity on the target RNA, and generally has good metabolic stability. Peptide nucleic acids are also commercially available for example from Applied Biosystems (Foster City, Calif., USA).

In another example of a modified nucleic acid molecule, the molecule comprises at least one morpholino phosphoroamidate nucleotide moiety. Molecules comprising morpholino phosphoroamidate nucleotide moieties are generally referred to as morpholino (MF) nucleic acids; see Heasman, Dev. Biol. 243, 209-214 (2002). Morpholino oligonucleotides are for example commercially available from Gene Tools LLC (Corvallis, Oreg., USA).

In a further example of a modified nucleic acid molecule, the molecule comprises at least one cyclohexene nucleotide moiety. Molecules comprising cyclohexene nucleotide moieties are generally referred to as cyclohexene nucleic acids (CeNA). See structure 10 in FIG. 1. Wang et al., J. Am. Chem. Soc. 122, 8595-8602 (2000), Verbeure et al., Nucleic Acids Res. 29, 4941-4947(2001).

Moreover, a modified nucleic acid molecule may comprise at least one tricyclo nucleotide moiety. Molecules comprising tricyclo nucleotide moieties are generally referred to as tricyclo nucleic acids (tcDNA); see Steffens et al., J. Am. Chem. Soc. 119, 11548-11549 (1997), Renneberg et al., J. Am. Chem. Soc. 124, 5993-6002 (2002).

Chimeric modified nucleic acid molecules containing a mixture of any of the moieties mentioned above can also obtained by methods known in the art, see for example, references cited above, and Wang et al, Proc. Natl. Acad. Sci. USA 96, 13989-13994 (1999), Liang et al., Eur. J. Biochem. 269, 5753-5758 (2002), Lok et al., Biochemistry 41, 3457-3467 (2002), and Damha et al., J. Am. Chem. Soc. 120, 12976-12977 (2002).

In yet another embodiment, caps can be attached to one end, both ends, and/or between the ends of the nucleic acid molecule in order to increase nuclease resistance of the modified or unmodified nucleic acid molecules described above to exonucleases. Any cap known to those in the art for increasing nuclease resistance can be employed. Examples of such caps include inverted nucleotide caps and chemical caps.

An inverted nucleotide cap refers to a 3'.to.5' sequence of nucleic acids attached to the nucleic acid molecule at the 5' and/or the 3' end. There is no limit to the maximum number of nucleotides in the inverted cap just as long as it does not interfere with binding of the nucleic acid molecule to its target mRNA. Any nucleotide can be used in the inverted nucleotide cap. Usually, the nucleotide cap is less than about forty nucleotides in length, preferably less than about thirty nucleotides in length, more preferably less than about twenty nucleotides in length, and even more preferably less than about ten nucleotides in length. Typically, the inverted nucleotide cap is one nucleotide in length. The nucleotide for the inverted cap is generally thymine, but can be any nucleotide such as adenine, guanine, uracil, or cytosine.

Alternatively, a chemical cap can be attached to the 5' end, to the 3' end, to both ends of the nucleic acid molecule, and/or to any moiety(ies) between the 5' end and 3' end of the modified nucleic acid molecule in order to increase nuclease resistance to exonucleases and/or endonucleases. The chemical cap can be any chemical group known to those in the art for increasing nuclease resistance of nucleic acid molecules. Examples of such chemical caps include hydroxyalkyl or aminoalkyl groups. Hydroxyalkyl groups are sometimes referred to as alkyl glycoyl groups (e.g., ethylene glycol). Aminoalkyl groups are sometimes referred to as amino linkers.

The alkyl chain in the hydroxyalkyl group or aminoalkyl groups can be a straight chain or branched chain. The minimum number of carbon atoms present in the alkyl chain is one, preferably at least two, and more preferably at least about three carbon atoms. The maximum number of carbon atoms present in the alkyl chain is about eighteen, preferably about sixteen, and more preferably about twelve. Typical alkyl groups include methyl, ethyl, and propyl. The alkyl groups can be further substituted with one or more hydroxyl and/or amino groups. Amino linkers are, for example, commercially available from TriLink Biotechnologies, San Diego, Calif.

As mentioned above, the nucleic acid molecule as defined and disclosed herein is useful in the treatment of fibrosis. The term "fibrosis" is well known in the art. As used herein the term "fibrosis," is to be understood as the formation or development of excess fibrous connective tissue in an organ or tissue as a reparative or reactive process, as opposed to a formation of fibrous tissue as a normal constituent of an organ or tissue. Particularly preferred herein is the treatment of cardiac fibrosis, in particular cardiac diseases that involve fibrosis, such as heart failure, cardiac hypertrophy, heart-related storage diseases and the like..

Exemplary fibrotic diseases to be treated in context of the present invention are, besides cardiac diseases involving myocardial fibrosis, also pulmonary, renal, hepatic, skeletal muscle or radiation-induced fibrosis.

Preferably, cardiac diseases are to be treated. Cardiac diseases are, for example, diseases like heart failure, cardiac hypertrophy, heart-related storage disease (e.g. M. Fabry), cardiomyopathies (such as dilated cardiomyopathy, hypertrophic cardiomyopathy with and without obstruction, restrictive cardiomyopathy, arrhythmogenic right ventricular cardiomyopathy and other forms of cardiomyopathy, like e.g. diabetic cardiomyopathy), dilatative or constrictive pericarditis, coronary artery disease, acute myocardial infarction, chronic myocardial infarction, cardiac arrhythmia, myocarditis-related fibrosis or heart valve disease, wherein heart failure is preferred. Also envisaged in this context is the treatment of cardiac hypertrophy, hypertensive heart disease, heart valve disease that may lead to valve stenosis or insufficiency (e.g. sclerosis), e.g. mitral valve stenosis and/or insufficiency, aortic valve stenosis and/or insufficiency, tricuspidal valve stenosis and/or insufficiency, pulmonary valve stenosis and/or insufficiency.

Non-limiting examples of heart failure are hypertensive heart failure, diastolic heart failure, (acute or chronic) right heart failure or systolic heart failure.

As mentioned also other diseases involving fibrosis (= fibrosis related diseases), not related to the cardiac system are to be treated in accordance with the present invention, e.g. pulmonary, renal, hepatic, skeletal muscle or radiation-induced fibrosis.

Non-limiting examples are lung fibrosis, chronic obstructive lung diseases, pulmonary hypertension, liver fibrosis due to a toxic surrounding, hepatitis, skin fibrosis, e.g. development of keloids after injury, blood vessel-related diseases, such as arterial stiffness related to age or hypertension, mediasclerosis, artherosclerosis, age-related fibrosis of different organs, gut sclerosis, e.g. during Crohn's disease, systemic sclerosis and CREST syndrome etc., kidney fibrosis, neoplastic fibrosis and/or rheumatoid arthritis.

Further well-known fibrosis-related diseases and disorders are e.g. endomyocardial fibrosis and idiopathic myocardiopathy, cirrhosis which can result from fibrosis of the liver, idiopathic pulmonary fibrosis of the lung, diffuse parenchymal lung disease, mediastinal fibrosis, myelofibrosis, post-vasectomy pain syndrome, retroperitoneal fibrosis and nephrogenic systemic fibrosis.

miR-223 is also useful in the diagnosis of fibrosis, in particular cardiac fibrosis. As shown in Fig. 3., the expression of miR-223 drastically decreased in failing cardiac fibroblasts. Accordingly, a method of diagnosing fibrosis and corresponding means are provided herein.

The present invention relates therefore to a method of diagnosing in a subject/patient suspected of suffering from fibrosis or suspected of being prone to suffering from fibrosis comprising the steps of

a) determining in a cell or tissue sample obtained from said subject/patient the expression level of miR-223; and
b) comparing the expression level of miR-223 determined in a) with a reference expression level of miR-223 determined in (a sample from) a control subject/patient (healthy subject),

wherein said fibrosis is diagnosed when said activity determined in a) differs from said reference activity.

Also a diagnostic kit useful for carrying out the above method is provided In a preferred embodiment, said kit comprises oligonucleotides or polynucleotides capable of detecting the expression level of miR-223.

For example, said kit may comprise (a) compound(s) required for specifically determining the expression level of miR-223. In a preferred embodiment, the kit (to be prepared in context) of this invention is a diagnostic kit.

In a particularly preferred embodiment of the present invention, the kit (to be prepared in context) of this invention or the methods and uses of the invention may further comprise or be provided with (an) instruction manual(s). For example, said instruction manual(s) may guide the skilled person (how) to determine expression level of miR-223, i.e. (how) to diagnose the fibrosis. Particularly, said instruction manual(s) may comprise guidance to use or apply the herein provided methods or uses.

The kit (to be prepared in context) of this invention may further comprise substances/chemicals and/or equipment suitable/required for carrying out the methods and uses of this invention. For example, such substances/chemicals and/or equipment are solvents, diluents and/or buffers for stabilizing and/or storing (a) compound(s) required for specifically determining the expression level of miR-223

The following relates to pharmaceutical compositions which may comprise the nucleic acid molecule described and defined herein above.

The pharmaceutical composition will be formulated and dosed in a fashion consistent with good medical practice, taking into account the clinical condition of the individual patient, the site of delivery of the pharmaceutical composition, the method of administration, the scheduling of administration, and other factors known to practitioners. The "effective amount" of the pharmaceutical composition for purposes herein is thus determined by such considerations.

The skilled person knows that the effective amount of pharmaceutical composition administered to an individual will, inter alia, depend on the nature of the compound. For example, if said compound is a an nucleic acid molecule the total pharmaceutically effective amount of pharmaceutical composition administered parenterally per dose will be in the range of about 1 μg /kg/day to 100 mg /kg/day of patient body weight, although, as noted above, this will be subject to therapeutic discretion. More preferably, this dose is at least 0.01 mg kg/day, and most preferably for humans between about 0.01 and 1 mg kg/day. The presently recommended dose for nucleic acid molecules lies in a range of between 8 and 80 mg per/kg/day. However, this dose may be further decreased subject to therapeutic discretion, in particular if concomittantly certain lipids are applied or if the nucleic acid molecule is subject to certain chemical modifications. If given continuously, the pharmaceutical composition is typically administered at a dose rate of about 1 μg/kg/hour to about 40 μg/kg/hour, either by 1-4 injections per day or by continuous subcutaneous infusions, for example, using a mini-pump. An intravenous bag solution may also be employed. The length of treatment needed to observe changes and the interval following treatment for responses to occur appears to vary depending on the desired effect. The particular amounts may be determined by conventional tests which are well known to the person skilled in the art.

Also envisaged herein is the application of the herein provided nucleic acid molecule using stents. A preferred application form is a drug eluting stent system. This system may be a polymer based drug delivering system or a polymer coated drug delivering system. It is to be understood that the nucleic acid molecule described and provided herein, is applied to the drug delivering system in combination with (a) polymer(s). Therefore the drug component (the active ingredient) is embedded in a non-erodible polymer carrier (base coat formulation) which is surrounded by a suitable topcoat layer to control the release of the embedded drug. A possible application form would be a system containing parylene C and the following two non-erodible polymers: polyethylene-co-vinyl acetate (PEVA) and poly n-butyl methacrylate (PBMA). A combination of the two polymers (67%/33%) mixed with nucleic acid molecule, makes up the basecoat formulation which is applied to a parylene C treated stent. A drug-free topcoat of PBMA polymer is applied to the stent surface to control the release kinetics of the nucleic acid molecule. Alternatively a single layer polymer e.g. a Translute® polymer carrier, might be used as drug delivering matrix. The drug/polymer coating is adhered to the entire surface (i.e., luminal and abluminal) of the stent.

Pharmaceutical compositions of the invention may be administered orally, rectally, parenterally, intracisternally, intravaginally, intraperitoneally, topically (as by powders, ointments, drops or transdermal patch), bucally, or as an oral or nasal spray. Preferably the pharmaceutical compositions of the invention are administered parenterally.

Pharmaceutical compositions of the invention preferably comprise a pharmaceutically acceptable carrier. By "pharmaceutically acceptable carrier" is meant a non-toxic solid, semisolid or liquid filler, diluent, encapsulating material or formulation auxiliary of any type. The term "parenteral" as used herein refers to modes of administration which include intravenous, intramuscular, intraperitoneal, intrasternal, subcutaneous and intraarticular injection and infusion.

The pharmaceutical composition is also suitably administered by sustained release systems. Suitable examples of sustained-release compositions include semi-permeable polymer matrices in the form of shaped articles, e.g., films, or mirocapsules. Sustained-release matrices include polylactides (U.S. Pat. No. 3,773,919, EP 58,481), copolymers of L-glutamic acid and gamma-ethyl-L-glutamate (Sidman, U. et al., Biopolymers 22:547-556 (1983)), poly (2-hydroxyethyl methacrylate) (R. Langer et al., J. Biomed. Mater. Res. 15:167-277 (1981), and R. Langer, Chem. Tech. 12:98-105 (1982)), ethylene vinyl acetate (R. Langer et al., Id.) or poly-D-(-)-3-hydroxybutyric acid (EP 133,988). Sustained release pharmaceutical composition also include liposomally entrapped compound. Liposomes containing the pharmaceutical composition are prepared by methods known per se: DE 3,218,121; Epstein et al., Proc. Natl. Acad. Sci. (USA) 82: 3688-3692 (1985); Hwang et al., Proc. Natl. Acad. Sci. (USA) 77:4030-4034 (1980); EP 52,322; EP 36,676; EP 88,046; EP 143,949; EP 142,641; Japanese Pat. Appl. 83-118008; U.S. Pat. Nos. 4,485,045 and 4,544,545; and EP 102,324. Ordinarily, the liposomes are of the small (about 200-800 Angstroms) unilamellar type in which the lipid content is greater than about 30 mol. percent cholesterol, the selected proportion being adjusted for the optimal therapy.

For parenteral administration, the pharmaceutical composition is formulated generally by mixing it at the desired degree of purity, in a unit dosage injectable form (solution, suspension, or emulsion), with a pharmaceutically acceptable carrier, i.e., one that is non-toxic to recipients at the dosages and concentrations employed and is compatible with other ingredients of the formulation.

Generally, the formulations are prepared by contacting the components of the pharmaceutical composition uniformly and intimately with liquid carriers or finely divided solid carriers or both. Then, if necessary, the product is shaped into the desired formulation. Preferably the carrier is a parenteral carrier, more preferably a solution that is isotonic with the blood of the recipient. Examples of such carrier vehicles include water, saline, Ringer's solution, and dextrose solution. Non aqueous vehicles such as fixed oils and ethyl oleate are also useful herein, as well as liposomes. The carrier suitably contains minor amounts of additives such as substances that enhance isotonicity and chemical stability. Such materials are non-toxic to recipients at the dosages and concentrations employed, and include buffers such as phosphate, citrate, succinate, acetic acid, and other organic acids or their salts; antioxidants such as ascorbic acid; low molecular weight (less than about ten residues) (poly)peptides, e.g., polyarginine or tripeptides; proteins, such as serum albumin, gelatin, or immunoglobulins; hydrophilic polymers such as polyvinylpyrrolidone; amino acids, such as glycine, glutamic acid, aspartic acid, or arginine; monosaccharides, disaccharides, and other carbohydrates including cellulose or its derivatives, glucose, manose, or dextrins; chelating agents such as EDTA; sugar alcohols such as mannitol or sorbitol; counterions such as sodium; and/or nonionic surfactants such as polysorbates, poloxamers, or PEG.

The components of the pharmaceutical composition to be used for therapeutic administration must be sterile. Sterility is readily accomplished by filtration through sterile filtration membranes (e.g., 0.2 micron membranes). Therapeutic components of the pharmaceutical composition generally are placed into a container having a sterile access port, for example, an intravenous solution bag or vial having a stopper pierceable by a hypodermic injection needle.

The components of the pharmaceutical composition ordinarily will be stored in unit or multidose containers, for example, sealed ampoules or vials, as an aqueous solution or as a lyophilized formulation for reconstitution. As an example of a lyophilized formulation, 10-ml vials are filled with 5 ml of sterile-filtered 1% (w/v) aqueous solution, and the resulting mixture is lyophilized. The infusion solution is prepared by reconstituting the lyophilized compound(s) using bacteriostatic Water-for-Injection.

The nucleic acid molecules may be delivered as follows: for example, the nucleic acid molecules can be injected directly into a cell, such as by microinjection. Alternatively, the molecules can be contacted with a cell, preferably aided by a delivery system. Useful delivery systems include, for example, liposomes and charged lipids. Liposomes typically encapsulate oligonucleotide molecules within their aqueous center. Charged lipids generally form lipid-oligonucleotide molecule complexes as a result of opposing charges.

These liposomes-oligonucleotide molecule complexes or lipid-oligonucleotide molecule complexes are usually internalized in cells by endocytosis. The liposomes or charged lipids generally comprise helper lipids which disrupt the endosomal membrane and release the oligonucleotide molecules.

Other methods for introducing nucleic acid molecules into a cell include use of delivery vehicles, such as dendrimers, biodegradable polymers, polymers of amino acids, polymers of sugars, and oligonucleotide-binding nanoparticles. In addition, pluoronic gel as a depot reservoir can be used to deliver the anti-microRNA oligonucleotide molecules over a prolonged period. The above methods are described in, for example, Hughes et al., Drug Discovery Today 6, 303-315 (2001); Liang et al. Eur. J. Biochem. 269 5753-5758 (2002); and Becker et al., In Antisense Technology in the Central Nervous System (Leslie, R. A., Hunter, A. J. & Robertson, H. A., eds), pp.147-157, Oxford University Press.

Targeting ofnucleic acid molecules to a particular cell can be performed by any method known to those skilled in the art. For example, nucleic acid molecules can be conjugated to an antibody or ligand specifically recognized by receptors on the cell. For example, the ligand can be DDR2 (discoid domain receptor 2) expressed on fibrotic cells. Alternatively, an antibody to DDR2 (discoid domain receptor 2) can be employed.

In one embodiment, the present invention provides a method for treating fibrosis in a mammal in need thereof. Preferably, the mammal is a human. The method comprises administration into the mammal an effective amount of a nucleic acid molecule defined herein above comprising at least ten contiguous bases having a sequence of bases as shown in the sequence of miR-223 (SEQ ID NO 16). The effective amount is determined during pre-clinical trials and clinical trials by methods familiar to physicians and clinicians. Also disclosed and envisaged herein is the use of a nucleic acid molecule defined herein above comprising at least ten contiguous bases having a sequence of bases as shown in the sequence of miR-223 (SEQ ID NO 16) for the preparation of a pharmaceutical composition for treating fibrosis.

The terms "treatment", "treating" and the like are used herein to generally mean obtaining a desired pharmacological and/or physiological effect. The effect may be prophylactic in terms of completely or partially preventing fibrosis or symptom thereof and/or may be therapeutic in terms of partially or completely curing fibrosis and/or adverse effect attributed to fibrosis. The term "treatment" as used herein covers any treatment of a disease in a subject and includes: (a) preventing fibrosis from occurring in a subject which may be predisposed to fibrosis; (b) inhibiting fibrosis, i.e. arresting its development; or (c) relieving fibrosis, i.e. causing regression of fibrosis.

The nucleic acid molecule can be introduced into the mammal by any method known to those in the art. For example, the above described methods for introducing the nucleic acid molecule into a cell can also be used for introducing the molecules into a mammal.

It is envisaged herein that the above described and defined nucleic acid molecules can also be applied in combination with conventional therapies. For example, one or more additional pharpharmaceutical agents can be used. Non-limiting examples of additional pharmaceutical agents are diuretics (e.g. sprionolactone, eplerenone, furosemide), inotropes (e.g. dobutamine, milrinone), digoxin, vasodilators, angiotensin II converting enzyme (ACE) inhibitors (e.g. are captopril, enalapril, lisinopril, benazepril, quinapril, fosinopril, and ramipril), angiotensin II receptor blockers (ARB) (e.g. candesartan, irbesartan, olmesartan, losartan, valsartan, telmisartan, eprosartan), calcium channel blockers, isosorbide dinitrate, hydralazine, nitrates (e.g. isosorbide mononitrate, isosorbide dinitrate), hydralazine, beta-blockers (e.g. carvedilol, metoprolol), and natriuretic peptides (e.g. nesiritide).

An additional pharmaceutical agent may also enhance the body's immune system, and may, therefore, include low-dose cyclophosphamide, thymostimulin, vitamins and nutritional supplements (e.g., antioxidants, including vitamins A, C, E, beta-earotene, zinc, selenium, glutathione, coenzyme Q-10 and echinacea), and vaccines, e.g., the immunostimulating complex (ISCOM), which comprises a vaccine formulation that combines a multimeric presentation of antigen and an adjuvant.

The additional therapy can also be selected to treat or ameliorate a side effect of one or more pharmaceutical compositions of the present invention. Such side effects include, without limitation, injection site reactions, liver function test abnormalities, renal function abnormalities, liver toxicity, renal toxicity, central nervous system abnormalities, and myopathies. For example, increased aminotransferase levels in serum may indicate liver toxicity or liver function abnormality. For example, increased bilirubin may indicate liver toxicity or liver function abnormality.

Moreover, one or more pharmaceutical compositions of the present invention and one or more other pharmaceutical agents can be administered at the same time. The one or more pharmaceutical compositions of the present invention and one or more other pharmaceutical agents can also be prepared together in a single formulation.

The present invention is further described by reference to the following non-limiting figures and examples.

The Figures show:

**Figure 1. Involvement of microRNAs in the regulation of proliferation/cell death.**

Neonatal rat CFs were transfected with microRNA precursors or unspecific precursor control (Pre-ctrl.). As an additional control some cells were treated with 10% fetal bovine serum (FBS), a known pro-proliferative stimulus. Cells were analysed 72 h post transfection. Treatment with miRNAs leads to different responses. For example, transfection with miR-214 has a pro-proliferative effect compared to transfection with Pre-ctrl.-treated cells, whereas miR-223-treated cells exhibit an anti-proliferative phenotype.

**Figure 2. Thymidine incorporation after transfection with miR-223 precursor.**

Incorporation of thymidine during DNA synthesis is a classical parameter used to quantify cell proliferation. As expected, 5% FBS (positive control) induces a significant increase in cellular proliferation. Treatment with miR-223 leads to a significant decrease of thymidine incorporation compared transfection with unspecific precursor control (Pre-ctrl.). This experiment confirms the finding of Figure 1 that transfection with miR-223 leads to a anti-proliferative/pro-apoptotic effect.

**Figure 3. miR-223 expression level in isolated different cardiac cell types under non-failing and failing conditions.**

In non-failing cells, miR-223 is preferentially expressed in cardiac fibroblasts (CF) compared to cardiomyocytes (CM). This difference in expression is maintained during heart failure albeit the expression is massively decreased in both cell types.

**Figure 4. Level of IL6ST mRNA in fibroblasts (CFs) from non-failing and failing hearts.**

IL6ST mRNA expression level is increased in failing cells compared to non-failing ones.

**Figure 5. Modulation of IL6ST mRNA expression level after treatment with miR-223 precursor in neonatal rat cardiac fibroblasts.**

Transfection of miR-223 precursor leads to a significant decrease of the IL6ST mRNA expression in cardiac fibroblasts.

**Figure 6. Modulation of IL6DT mRNA expression level after treatment with a miR-223 inhbitor in neonatal rat cardiac fibroblasts.**

Transfection of miR-223 inhibitor leads to a dramatic increase of the IL6ST mRNA expression in cardiac fibroblasts. Taken together with the results from figure 5, these data clearly show that miR-223 is functional in cardiac fibroblasts and targets IL6ST.

**Figure 7.**

List of precursor sequences of "candidate microRNAs" which passed our microarray filtering criteria (predominant expression in CFs and dysregulation under pathological conditions) and which are conserved among human, mouse and rat. It is of note that the human mature miR-199 molecule has two different human precursor, namely miR-199a-1 and -2, from which the same mature microRNA miR-199 is transcribed

**Figure 8.**

List of " candidate mature microRNAs" which passed our microarray filtering criteria (predominant expression in CFs and dysregulation under pathological conditions) and which are conserved among human, mouse and rat.
The Examples illustrate the invention.

**Example 1:**

***Methods and results***

**Identification of the miRNA pattern in CFs**

Using a microarray-based analysis the microRNA expression signatures in different cardiac cell types and under different conditions (cells from normal compared to failing hearts) was determined. For the miRNA microarray assay (Exiqon, Denmark) RNA extracted from high-purity cell preparations from the myocardium (cardiomyocytes (CMs) vs. cardiac fibroblasts (CFs)) as well as whole myocardial tissue RNA were used. The murine model of heart failure used was a transgenic mouse line overexpressing the human betal adrenergic receptor compared to wild type mice. To our knowledge, this is the first time that such a microarray-based study was carried out on isolated cardiac cells instead of whole. To supplement these data, also hearts and isolated cells from neonatal rats were analysed.

The results of the miRNA pattern show that many microRNAs are deregulated during heart failure and that very different subsets are up- or down-regulated in CMs and CFs under these pathological conditions.

The number of dysregulated miRNAs in the heart failure model: Applying a signal difference of 0,4 units on the log-2 scale (i.e. a more than 31,5% upregulation or more than 24% downregulation) in cardiomyocytes roughly 100 upregulated and approx. 30 downregulated miRNAs were found). In fibroblasts, approx. 70 upregulated and 60 down-regulated microRNAs were observed.

Based on these findings a set of 14 "candidate microRNAs" was chosen, which are preferentially expressed in CFs compared to CMs, highly dysregulated in CFs in failing hearts, and which displayed a substantial fluorescence intensity in the microarray screen (to filter out those, which are only minimally expressed). The differential expression observed in the microarray study has been validated using realtime-PCR for a total of 5 microRNAs (data not shown). The realtime-PCR results show a high degree of reproducibility of the array results. The expression of the following 5 candidate microRNAs was retested and fully confirmed with qPCR: miR-142-5p, miR-223, miR-152. Regarding miR-690, miR455: dysregulation in fibroblasts in failing conditions was confirmed, regulation in myocardium, cardiomyocytes and cell-type specificity could not be confirmed in all cases.

In the following step the conservation of the candidate microRNAs among mammalian species was analyzed using the publicly available data base miRbase (www. mirbase.org). Only microRNAs (8 in total) which are substantially conserved among human, mouse and rat were accepted for further analysis (Fig. 8).

**Functional analysis of the CF preferentially expressed**

In a further step it was investigated in which general cellular function(s) the selected, deregulated mircoRNAs are involved. During the development of heart failure, CF transdifferentiate into myofibroblasts, which are hyperproliferative and secrete high amounts of ECM (esp. collagen) and signaling molecules. It was investigated whether the selected microRNAs show an effect in such a context. Accordingly, several *in vitro* assays were designed, which mimic these *in vivo* situation of heart failure. These in vitro assays are based on the transfection of neonatal rat CF with microRNA.

Furthermore, an experimental strategy was developed allowing simultaneous quantification of the following parameters: proliferation and collagen production.

For this experiment cultivated neonatal rat CFs were used. 24h after isolation 20,000 cells were seeded on black 96-well plates (Ibidi, Martinsried, Germany) containing NRCM medium with 1% FBS and cultured in a humidified 37 °C/1% $CO_2$ incubator for 24h. Afterwards, the cells were transfected with the appropriate human microRNA precursor (50 nM, Ambion, Austin, USA). Before transfection the medium was replaced with NRCM medium with 5% FBS without antibiotics. The transfection procedure using Lipofectamine™ 2000 (Invitrogen, Karlsruhe, Germany) was performed according to the manufacturer's instructions. After 4-6 h of incubation in a humidified 37 °C/1% $CO_2$ incubator the cell medium was exchanged to NRCM medium with 1% FBS. 72 h later, the medium was collected and stored at -20°C. Cells were incubated fixed through a 5 min incubation in 50 μl 4% paraformaldehyde (PFA) per well. Cells were then washed three times with 1x PBS at RT and kept at 4 °C.

Cellular proliferation was measured with automated microscopy (X-Cite$^R$120 illumination system, EXFO, Munich, Germany; SPOT PURSUIT CCD camera, Visitron Systems, Puchheim, Germany; BD Carv II, BD Biosciences, Heidelberg, Germany; AxioObserver.Zl, Zeiss, Göttingen, Germany). For this, nuclei were stained by application of DAPI (100 μg/μl; Sigma-Aldrich, Taufkirchen, Germany), diluted 1:100 in 1x PBS, at 37°C for 10 min. After three washing steps with 1x PBS, cells were covered with 100 μl of 50% glycerol solution per well and stored at 4 °C. In a computerised analysis (MetaMorph imaging software, Molecular Devices, Ismaning, Germany) cell numbers were counted in four different fields of view for each well.

Thus, it was determined, if a microRNA of interest has a pro- or anti-proliferative effect on CFs compared to cells treated with scrambled oligonucleotide controls. As a positive control for proliferation-inducing conditions cells were cultured in the presence of medium containing 10% FBS (Figure 1). The data shown in Fig. 1 demonstrate that transfection with miR-21, miR-199a-3p, miR-214, miR-455 and miR-665 increases the number of counted nuclei. Thus, these microRNAs act in a pro-proliferative (or anti-apoptotic) manner in cardiac fibroblasts. Transfection with miR-223 reduces the number of counted nuclei and appears therefore as a anti-proliferative (or pro-apoptotic) modulator.

**miR-223: a miRNA inducing fibrotic actions in CFs during the development of cardiac fibrosis**

*miR-223 acts as an anti-proliferative factor in CFs*

During heart failure, CFs are proliferative and hypersecretive. It is well established that in the model of heart failure (beta-1 transgenic mouse model of heart failure; see Engelhardt S, Hein L, Wiesmann F, Lohse MJ. Progressive hypertrophy and heart failure in betal-adrenergic receptor transgenic mice. Proceedings of the National Academy of Sciences of the United States of America. Jun 8 1999;96(12):7059-7064.) which is used here murine hearts undergo cardiac fibrosis.

Our *in vitro* observations show surprisingly that the overexpression miR-223 in CFs has an anti-proliferative/pro-apopotic effect on neonatal rat cardiac fibroblasts (Figure 2).

Next, we determined the expression level of miR-223 with quantitative PCR in isolated cardiac cell types (CFs vs. CMs) from healthy and failing murine cells using a commercial assay (Applied biosystems), in order to confirm our microarray data. "healthy" refers here to cells isolated from non-transgenic controls, "failing" refers here to the beta-1 adrenergic mouse model.

Indeed, miR-223 is predominantly expressed in CF compared to CMs irrespective of the cardiac status. Furthermore, its expression in CFs dramatically decreases during heart failure (Figure 3). These results are concordant with the anti-proliferative effect observed in vitro in neonatal rat CF. Without being bound by theory, it is therefore believed that the loss of miR-223 in CFs during the development of heart failure acts as a trigger which induces the proliferation of these cells in cardiac fibrosis.

**Identification of a putative mRNA target for miR- 223 in CFs**

Several microRNA target prediction algorithms (TargetScan, miRanda, picTar) were employed to identify putative targets for miR-223. It was found that the level of one mRNA (IL6ST), whose 3'-UTR contains a miR-223 binding site predicted by all three algorithms, is highly upregulated in failing CF **(Figure 4),** a fact that corresponds very well to the decrease of miR-223 expression under these conditions **(Figure 3).**

Furthermore, it is demonstrated that upon the transfection with miR-223 precursors the expression of IL6ST mRNA in rat CF is significantly decreased **(Figure 5).** In turn, inhibiting miR-223 by transfecting a specific inhibitor (Exiqon) leads to an increase of the mRNA level of this gene **(Figure 6).** These results indicate that miR-223 is very likely involved in the regulation of IL6ST expression. Moreover, these results confirm that miR-223 is functional in CF. a luciferase-based assay was employed in order to further support the notion that IL6ST is a direct target of miR-223. The 3'UTR of IL6ST was cloned behind a luciferase reporter gene using the pmiR-REPORT System (Ambion). After transfection of miR-223, a decrease of the luciferase activity was observed. This indicates that miR-223 binds to the 3'UTR of IL6ST and thus inhibits translation of the luciferase gene.

***Conclusion***

It is shown herein that a miRNA, miR-223, is predominantly expressed in CFs, deregulated in this cell type in failing hearts, and that it posesses an anti-proliferative effect. This microRNA appears to target IL6ST next to other target mRNAs. As shown herein above, this anti-proliferative effect can be observed upon transfection of miR-223 into CFs. Thus, it is demonstrated that miR-223 can be used in the treatment of fibrosis, in particular in order to prevent the development of cardiac fibrosis.

The present invention refers to the following nucleotide and amino acid sequences:

The sequences provided herein are available in the "miRbase" data base and can be retrieved from www.mirbase.org (University of Manchester); Theses sequences also relate to annotated and modified sequences. The present invention also provides techniques and methods wherein homologous sequences, and variants of the concise sequences provided herein are used. Preferably, such "variants" are genetic variants.

SEQ ID No. 1:
Nucleotide sequence of human precursor miR-21 (Accession No. M10000077).

UGUCGGGUAGCUUAUCAGACUGAUGUUGACUGUUGAAUCUCAUGGCAACACCAG
UCGAUGGGCUGUCUGACA

SEQ ID No. 2:
Nucleotide sequence of human precursor miR- 142-5p (Accession No. M10000458).

GACAGUGCAGUCACCCAUAAAGUAGAAAGCACUACUAACAGCACUGGAGGGUGU
AGUGUUUCCUACUUUAUGGAUGAGUGUACUGUG

SEQ ID No. 3:
Nucleotide sequence of human precursor miR-152 (Accession No. M10000462).

UGUCCCCCCCGGCCCAGGUUCUGUGAUACACUCCGACUCGGGCUCUGGAGCAGUC
AGUGCAUGACAGAACUUGGGCCCGGAAGGACC

SEQ ID No. 5:
Nucleotide sequence of human precursor miR-199a-1 (Accession No. M10000242).

GCCAACCCAGUGUUCAGACUACCUGUUCAGGAGGCUCUCAAUGUGUACAGUAGUC
UGCACAUUGGUUAGGC

SEQ ID No. 6:
Nucleotide sequence of human precursor miR-199a-2 (Accession No. M10000281).

AGGAAGCUUCUGGAGAUCCUGCUCCGUCGCCCCAGUGUUCAGACUACCUGUUCAG
GACAAUGCCGUUGUACAGUAGUCUGCACAUUGGUUAGACUGGGCAAGGGAGAGC
A

SEQ ID No. 7:
Nucleotide sequence of human precursor miR-214 (Accession No. MI0000290).

GGCCUGGCUGGACAGAGUUGUCAUGUGUCUGCCUGUCUACACUUGCUGUGCAGA
ACAUCCGCUCACCUGUACAGCAGGCACAGACAGGCAGUCACAUGACAACCCAGCC
U

SEQ ID No. 8:
Nucleotide sequence of human precursor miR-223 (Accession No. MI0000300).

CCUGGCCUCCUGCAGUGCCACGCUCCGUGUAUUUGACAAGCUGAGUUGGACACUC
CAUGUGGUAGAGUGUCAGUUUGUCAAAUACCCCAAGUGCGGCACAUGCUUACCA
G

SEQ ID No. 9:
Nucleotide sequence of human precursor miR-455 (Accession No. MI0003513).

UCCCUGGCGUGAGGGUAUGUGCCUUUGGACUACAUCGUGGAAGCCAGCACCAUGC
AGUCCAUGGGCAUAUACACUUGCCUCAAGGCCUAUGUCAUC

SEQ ID No. 10:
Nucleotide sequence of human precursor miR-665 (Accession No. MI0005563).

UCUCCUCGAGGGGUCUCUGCCUCUACCCAGGACUCUUUCAUGACCAGGAGGCUGA
GGCCCCUCACAGGCGGC

SEQ ID No. 11:
Nucleotide sequence of human mature miR-21 (Accession No. MIMAT0000076). UAGCUUAUCAGACUGAUGUUGA
SEQ ID No. 12:
Nucleotide sequence of human mature miR-142-5p (Accession No. MIMAT0000433).
CAUAAAGUAGAAAGCACUACU
Sr Q ID No. 13:
Nucleotide sequence of human mature miR-152 (Accession No. MIMAT0000438).
UCAGUGCAUGACAGAACUUGG
SEQ ID No. 14:
Nucleotide sequence of human mature miR-199a-3p (Accession No. MIMAT0000232).
ACAGUAGUCUGCACAUUGGUUA
SEQ ID No. 15:
Nucleotide sequence of human mature miR-214 (Accession No. MIMAT0000271).
ACAGCAGGCACAGACAGGCAGU
SEQ ID No. 16:
Nucleotide sequence of human mature miR-223 (Accession No. MIMAT0000280).
UGUCAGUUUGUCAAAUACCCCA
SEQ ID No. 17:
Nucleotide sequence of human mature miR-455 (Accession No. MIMAT0004784).
GCAGUCCAUGGGCAUAUACAC
SEQ ID No. 18:
Nucleotide sequence of human mature miR-665 (Accession No. MIMAT0004952).
ACCAGGAGGCUGAGGCCCCU
SEQ ID No. 19:
Nucleotide sequence of the human mature miR-223 seed sequence.
GUCAGUU

The following shows coding sequence of the miRNA precursors sequences depicted and described herein above.
Hsa miR-21

ENST00000362134
chromosome:GRCh37:17:57918027:57919298:1

TCCACCTACAACAAGAATTTCTTAAGCTTTCTTTTATTTGCATGAGAGAGCCACTACC
AA
GGCATGTTTTGTTATGCTGAAACTGGGCTGCTGCATACTGCTAAATGGCACCTCTGG
GAT
TGGCCTACCTGGGGATTTCTTGGTTTGTGAAAACAGGAGAGGAGAAATATCTCATAC
AAG
TGAAAGGATACTGGAGAGAGAAATTACCCATTTCTAAAAAAAAACCACACTCTGTC
GTAT
CTGTGTTAATGTTTTCTAGCATGTACTCTGGTTTCAACAGACACAAATTTATATGTTA
AC
CCAGTTTTCTTGCCGTTCTGTAAGTGTTTTATTCTTAGTGTGATTTTTTTCCATTGGGA
T
GTTTTTGATTGAACTTGTTCATTTTGTTTTGCTTGGGAGGAAAATAAACAATTTTACT
TT
TTTCCTTTAGGAGCATTATGAGCATTATGTCAGAATAGAATAGAATTGGGGTTCGAT
CTT
AACAGGCCAGAAATGCCTGGGTTTTTTTGGTTTGTTTTTGTTTTTGTTTTTTTATCAAA
T
CCTGCCTGACTGTCTGCTTGTTTTGCCTACCATCGTGACATCTCCATGGCTGTACCAC
CT
TGTCGGGTAGCTTATCAGACTGATGTTGACTGTTGAATCTCATGGCAACACCAGTCG
ATG

GGCTGTCTGACATTTTGGTATCTTTCATCTGACCATCCATATCCAATGTTCTCATTTA
AA
CATTACCCAGCATCATTGTTTATAATCAGAAACTCTGGTCCTTCTGTCTGGTGGCACT
TA
GAGTCTTTTGTGCCATAATGCAGCAGTATGGAGGGAGGATTTTATGGAGAAATGGG
GATA
GTCTTCATGACCACAAATAAATAAAGGAAAACTAAGCTGCATTGTGGGTTTTGAAA
AGGT
TATTATACTTCTTAACAATTCTTTTTTCAGGGACTTTTCTAGCTGTATGACTGTTACT
T
GACCTTCTTTGAAAAGCATTCCCAAAATGCTCTATTTTAGATAGATTAACATTAACC
AAC
ATAATTTTTTTAGATCGAGTCAGCATAAATTTCTAAGTCAGCCTCTAGTCGTGGTTC
AT
CTCTTTCACCTGCATTTTATTTGGTGTTTGTCTGAAGAAAGGAAAGAGGAAAGCAAA
TAC
GAATTGTACTATTTGTACCAAATCTTTGGGATTCATTGGCAAATAATTTCAGTGTGGT
GT
ATTATTAAATAGAAAAAAAAAATTTTGTTTCCTAGGTTGAAGGTCTAATTGATACGT
TTG
ACTTATGATGAC

Hsa miR-142-5p
ENST0000038435
>chromosome:GRCJ37:17:56407993:56409279:-1

GAGGCTGGGAATTGAGGCCCTGGTGTGTTCAAGACCTTGGGCTGGTGGGGAGGCCA
GGGC
CTGAGGCCCTGGGGGGTCAGGACCCTGGGCTGTTGGGGAGGCTGGGGACTTAGGCC
CTGG
GGGGTCAGGATCTTGGGCTGGTGGGGAGGCTGAAGGGTGGGGACTGAGGCTCTGGG
CAGT
CAGGACCTCACGGTTGGGGTGGACTGGAGACCAAGACCTTGGCAGGGGAGCTGTGG
CTGC

CTCATTTGGACGCTGGAGGGTGGCTAGCGTGGCTGGAAGCGGCCAGCCAGGGGTTC
ACAG
AACTGAAGGTGAGGCCTCCAGAGGCCCTAGTCTCTACCTGAGTGTCTCTGAAACTGG
GGG
GATGGGGTGGAGCCTTTAGGGGGAAGGGAAGAGGGAACTGAAGAGGAAGTGGGGG
AGGGA
GGTAGAGGAGGCAAGTCTGGCGCCATGCTGAGTCACCGCCCACAAGGCCCAGGGCG
GGCC
CTCGGGGGGCCCTGGCAGGGTTGGGGGGATCTTAGGAAGCCACAAGGAGGGCTGGG
GGGC
TCTTGGAGCAGGAGTCAGGAGGCCTGGGCAGCCTGAAGAGTACACGCCGACGGACA
GACA
GACAGTGCAGTCACCCATAAAGTAGAAAGCACTACTAACAGCACTGGAGGGTGTAG
TGTT
TCCTACTTTATGGATGAGTGTACTGTGGGCTTCGGAGATCACGCCACTGCTGCCGCC
CGC
TGCCCGCCACCATCTTCCTCGGCGCTCGGGGACCTCGTGTGACAGGTGAGCACCTTA
CGG
CCCCTCCCTACCCTGCCCAGATGCCTGAAAGGCCTCCATGGCTTTCCTGCCCTTCCTG
GT
TCCGGACAGCTGGGGAAAGGCCACAGCAGCTCCTCTGCTGCCCTGCAGTCTTTGGGG
GCG
GGGAGGGCTGGACATGTGGAACCCTGATGCAGCCGCAGCGTCAAGGACGAGGAAG
GGGTG
GGAAGGGATGGTACGTGGAGGGGAATGGGTGGTGGGACCAGGGACCCAATGCTAA
TAAAG
ACTGGACTGTGCTTCTCTTTTGTCTGAGACTCTGTTGCTGGGGTGGGGAGCTGAATG
CGG
GCGGCAGGCACATGGACAGTATGGGCGCACAGAAGGGCTGGGAGGGAGAACCTCT
GTCGC
TCCTCCAGAAGATACGGGTTTGGCCCTGCCTATGAGATCAGAAGAGGGCTCTGGGC
CTGG

CCAGGCCACAGCCGGAGGAGAGAAGGGCCTGGGCCATCTGCAGAAAGGGGCAGGG
ATGTA
AGGCATCCTCTCACCTTCTACCCTAGC

Hsa miR-152
ENST00000385212
chromosome: GRCh37:17:46113927:46115213:-l

GGGGCGGCGCGGAGGCGGGGCGGCGGCGGCGGGCAGGCGGCGGGCGGCGCTGTCA
GTGCG
AGGCGGCGAGCGGAATGCAGCGGCCCGAGGCTGGCCACGTCCGCACCCGGGGGAG
GGGGC
CGCGGCGGCAGGCCGGGGGCCGGCGCCGCCTGCTCGAGCCGGGGAGCCCTCGGGGC
TGCG
GGTACGGAGCGGGCGGGCGCGGGGATCCCGGGCGGCGGGAGCTGGGCAGGGACAG
AGCAG
GGTTAGGGGGAGCAGCCAACTCAGAACTCGCGCGCCCGGCGGCTGGGAACTTTGTG
TCAC
CCCTGACTGGCCCCAGGACCCGGGAGGAAAAGTTCGTCCCAGCGCCGACCGGAGAG
GAGG
CCTGTCCTGAGTCCGTCGGCGCGGGGCTGAAGTTCTGGGTCCGTTTGGAGTGGGGGT
GGC
GGGTGAGTTGCGATTCCGCGGGGAGGGTAAGGAGTGGCCTGTCCGTCCCAGACTCG
GCTC
CCATCACCGGTGGGTGATGGGCATGCTTCTGGAGTCTACCCGGGCCAGGGCCTGGG
GGCG
CGCTGATAGCGCAGGTCCAGCCCGGCCAGGGATCAGCTGGAAGAAGGAGGCTCGGC
CCGC
TGTCCCCCCCGGCCCAGGTTCTGTGATACACTCCGACTCGGGCTCTGGAGCAGTCAG
TGC
ATGACAGAACTTGGGCCCGGAAGGACCTTCTGCACCCAACGGGCACAGCGCCCACT
CGGG
GCCTGCAGTGGAACATCTGCCTGGGAGTGGAGTGGGCACCTGGGTTGGCCCATGTG
GCAC

AGGGCTGGGCAGAACCAGCTGTGGACCTTGAGGACTGGGGTTGCTGGGGCCTGAGG
ATGT
ATCCTAGGGCCAGCAGATCCCATAGACACAGGGTGTCCACCTCGAGTCAGGGGCCA
AACG
GAACTCTCCTCTCCGGTGTCAGTTGCAGGAACCTTCCCTCTACACCATCAAGGCTGT
TTT
CATCCTAGATAATGACGGGCGCCGGCTGCTGGCCAAGGTAACCTCTGACCCCACCC
ATGA
GGGACACAGTTCCCACTCAGCTGACACTCCTGTCTCAGGAGAAACTCCACTGTGATC
TCT
GTGACTCAGGGCAGCTCAGCTTAGACCCCTCCAGAGGTCCTTCTCATCCTCCTGCCT
TGT
CAAGGTGCTGCTCCCTGAAATGAGGGGTCTCCTCTATTTCTACTCATGTTCCCTGAA
ATG
TTTCCTTATCATCCTGCTACTGGCCAGTTGGACCTGGGCATGGGAAGGTTCTGCCTA
ATG
GGTCAGTGGAGTGTTGAGCCTGGGACC

has-miR-199a-1
ENST00000385019
>chromosome:GRCh37:19:10927502:10928772:-1

CTCCCACCTTTCGGGGAAGAGTTTTGGGGGCTTTAGAAGGGGCTAGGGGAGGGGGC
TCTG
CAGAAGCCCTGCCCCCGGCTCGGACGTTTGCCCAAGGGCCTTTGCTGCCCTGACCCT
ATC
ATATCACGCGGCCGGAAGCTTCCAGCCAGCAGGGCCAAGTAACTGAGTCACAGGCC
ATTC
CAGCTAATGCCACATCTGGAACTGTTTACAGTGCGATTCCGCCGAGAAATCAGTGGC
CGC
CTTCCTGGTGCTGCCGGCACGCACGCGTGCGCGCGCGCACACACACACACACAC
GTGT
GTGTGCGCCCCTCCTCCCCACCCCCGACCCCCAAAGAGTCAGACATTCCTCCTGAGC
CCA

GAAGCCACGATCCCAAACCCTGCCTCCTGCTCCGCCTCCCCCACTCTTTAGGATTTCC
TG
AAAACCCAGAACTTTCTCCAGATGCGAGCCGGCCCCAGCCTTGCCACGTCAGAAGG
GACA
GAGCGGATCGTCTCGGGAAGAGTGGTGGTTTCCTTGGCTGCTCAGAGGTGCTGAGG
CACG
GCCTGGCCTGGTGGCCCCAGCGTCTGCCTGGGGGGTTCTGCAGGATGGATAGCCGG
CCCC
GCCAACCCAGTGTTCAGACTACCTGTTCAGGAGGCTCTCAATGTGTACAGTAGTCTG
CAC
ATTGGTTAGGCTGGGCTTGGGTGAGCGGCTCGTCGAGACAGGCCCCCCAAACTCGC
CGGC
AGGTGAGTGTCATTTTCCACCACCCCGTTTCCCACTGTGGCAGAGCCTCGCATAGAA
GAT
TCGAGGGCCTGGGTGGGAGAAGAGGCACTGGGGAAAGGCAGAGGGAGCCCCGAGG
CCGGC
CGAGGGGTTGGAGGCCCTGGCCCTATCAGTCTGCCATCCCCACCCCATGGCTGTAAA
TGT
CTTGTTTATTTTTTAAATAAAGAGATATTGATGTCTTGTGTCTCACTGAGGCATCTAA
GA
GGGTGGCCTCCTCCTCAGGGGTGGCTGCCACGTGTGTGACTCCAAGGGTCCCTGCTG
GGA
TCCTAACTTGGGTGGTGGCGGGGGTCAGGTTCCATGGCTGCCCCCTCCTGAATGGAG
CCT
GCTTTGTGTCTGGGGCTCACACAGGGCCCCTTTCACAGGTCGGCTGTTTCTCATTACG
GC
CGGCGCCCGGCCCTTCTCCCACTGCTGCTCCAGACTCCAGTGTGGGGCTCCACGGGA
GGG
TCGGGGGCTCTGGCTTTCAACTCTCAGATGAGAAAAACACGAAGGGCCCCTCACGT
GAAG
GAAGAGGAGAT

has-miR-199a2
ENST00000385289
chromosome:GRCh37:1:172113075:172114384:-1

GCTCAAAAAGGTTTCCAAATGAGCTCTGCCTTCCAGTGTTCAACTTTCTGTTTACTAT
TT
CATCTACCCTCTATCTTCAAATTACTTCTCTAGCCTGGGTCAGACAGTAAATTTGTAC
TT
CTACACTAACTCACTTTCAGTTGGCCACAGCCGAACTGTACATCTTGACTCTTTTTTT
TT
TACAGCTTTTCCATACTGGGGCCCACTTCCTGCCCAGTTGAGGAAAAAAATCTGGCC
TGA
AGATGAAATGACTGCTTAACGTTACACTAAAACATCTGGTACCATTTTATGCACAGA
CCC
ATGTCTGAGAGCAGGCGATTCTAGCGGTCTCTCCAGCGGCACAGCGCATACCCAAA
CCTC
CCCAGGGTGACATCATCCCATATATGGACTCTCCAGCCCAGCCCTCCCCCTTTTCCTG
GT
CCTAAATTCATTGCCAGTTCCCCAGTCTGCAGCAAATGTGCCACGTCAAGACTGGAA
ATC
ACAGCCCTTGAGTGTGTCTCAGTCAGCGCAGCAGAATTCAGGGGGGAAAGCTGAAT
GCAA
CCCCTGGTGCAGGAAGGGAGGCTTTTCCTGAGGACCGGGAGAGGATTTTAAGTACA
TAGA
AGGAAGCTTCTGGAGATCCTGCTCCGTCGCCCCAGTGTTCAGACTACCTGTTCAGGA
CAA
TGCCGTTGTACAGTAGTCTGCACATTGGTTAGACTGGGCAAGGGAGAGCAACGCCA
TGGA
CCGCTGGGGACAAAATGGGCTGTTTCCAAGGAGAAGACATTTGTTTGCTCCTTTTTT
GAA
TCTCATATCCAGTGTTTTTCTTCACAGGTTTTGCACACTAGGGACCAAGAAGCCCTC
GGG
GACACTGCTGAGAAAAGACTGCCGAAGGGAAGACCAGCGGGTAAGGCCTTCTTGAC
TGCT

CACCTGGTCTGTGACCAAGGGAACCTAGGTGGGGAGCCCATGATTTGTGTTAACATG
CAA
GGGAAACAGAGGTACATGTGTGATGGGTAGAGATGTATTTAATTTTAAAAGAGCAA
AAGT
GTGTGGTATATAGCGATTTACTGTTTTTTTTTTTTTATTTCTGAAGATTGATAATTCA
A
GTGTTTTCTTAAAGGTTTTCCCACTTCAAAATGAAATATGAAAAAACTCATTCCAGA
CAC
TTAAGAATAGTTTAATGAGGCTACTTTTGAATTGTTTAATAATAATTCAAAGATTTA
AAA
ACTTATATGGATGAAGGCTGTATGTATAACTTTTAACATCCCAGGACTGAAGTCATT
TTT
CCCCAAAGTAAAGAATACTTGCTGGTTTTTAAATGCTATATGGATAGTGC

hsamiR-214
ENST00000385214
>chromosome:GRCh37:1:172107338:172108647:-1

TTTCACATATAATTGGTAAAAACATATTCCCTTGTAGGATCCTATTATCAGAGCATC
AAA

TACCAAAGTCAGCAACTTGGGTTGCCAAATAAATTAGGCTCATTAATTATTTTGAAA
AGG

GCTGCTGTTTCCATGTAAATGATAATTTTCTTTTTCATGGACAAGGTTATGGTACTTA
TC

TACTAAAAGAAACATGCATAAACATAGTTTTTATCTTTAACTTCAGTATTAAAATAT
GCA

GTTTTTTGCAAAGAGGAAAAACATCTGGTTCAATTACGCTGAACTCTGACTACATGT
GGG

CCAGTAATAATATGAATTGGACTTAAGAATAAACCTTGTGTTTAATCTCTTTTTTTCC
TT

AAAATTTTAATGTGAGTTTTCTGTTACGCAAATTATCCATGTTAGCACATTTGGAAC
AAA

TGTATAAATGTACTTTCTGAATAAAGTCAAAAGTCTAACTTGTTTTTAGTTCCATAAT
GT

TTTAATGTTTAATTATATTGTGTATTTTTCTCCCTTTCCCCTTACTCTCCAAAATCACC
A
AATCTGGAAAACAGGCTGATTGTATCTGTCTATGAGCAAAGGAAACCTGAAGGAAC
CAAG
GGCCTGGCTGGACAGAGTTGTCATGTGTCTGCCTGTCTACACTTGCTGTGCAGAACA
TCC
GCTCACCTGTACAGCAGGCACAGACAGGCAGTCACATGACAACCCAGCCTGAATGA
CAAC
CAGCCATTGAAAGAAAGCAGCCCTCACACCATAGCATCTACACCAAGAGCTACAAC
CAAA
ATGAGGGGCTCGGGGGCCTGGGGTTTTAATCTGTTGGTTTAATACTTAACTCATGTA
TTA
TTTTATTTTGATTCAAGTATTAAAACCCACCAAAAGTAAAGTGAGTGGTGCCTATGA
AAT
ATAATAAAACCCATTTCTGAAAATGTTCCTGTGCATTCTATAGTTCTCTTTTCCTAAT
TG
TTGTCAGCAAATCACTACCCTTGACCTTTATTGTACACAGCTTTGCAGCACCCTATAT
TG
ATATAAAAACTCAATATAACAAAACAATATAACTTCAGTCTGATGCTTAACAAAGA
ATAA
ATTATTCTAAGAATTCTTGTCCATAAACTAAGTATGGTTTTCTATGTCATGATTTTTT
TT
TTTTTTTTTTTTTTTTGGAAGGGGATAGGGTGGAAAGACAGAGAAAAGAAAAGGGA
GAAA
AAGAGTATTCCAAAGGATCAAGTGCTTTGAGTTAACTCTGCCAGTCTGGGTTAACTC
TGC
CAGTCTGGGTCTGGGCAAAACACAGTGAAAAGACTACTATTTCCAACTAG
.

Hsa miR-223
ENST00000385204
>chromosome:GRCh37:X:65238112:65239421:1

TTATCTCCTGTCATTCTCACAATAACCTTATGAGGTAGGTATTATACTTACATACATT TT

GTGGGTGTAGAAAACTTGAGCTCAGAGAGGTGAAATGATGTATCCAAGGTTGCCTA ACTA

GCTAATGGAAGAACTAGGATTTAAGCTCATGTTTAACCTCAAAGCCTATGTGCATAA CCA

CAACACTGAAAACACAGCTTGGGAGAATTGAGAAGAGGGAGCAAGGATGCTGACT CTAGG

GTCTGGTACTTTCCTCAAGTCAGGGAGGCAGGATGATGACCAAATTTTCCCCTCTCT GCT

GCATAGAGGAACCCACTGAAGCTTGGGACCCCTAGAAATGCCATAGCTACAGGACT CAGG

CCTGATGAGCTTCCAGCTGAGCACTGGGTGTCACTCGGGCTTTACCTGCTTATCTTCA GG

ATCTCTCTTCTGGTTAGGAACCCTATGCCTATTTTGCTCACTTCCCTATTCTGGTGCTT T

GGTTGGTCCTTTGAGCAAGATCCCCGGGGCTAAGGGTGTGACTTCATCATTCCTTTC TCT

CTCTTTCCCTCTAGGGTCACATCTCCCAGGAAGATCTCACTTCCCCACAGAAGCTCTT GG

CCTGGCCTCCTGCAGTGCCACGCTCCGTGTATTTGACAAGCTGAGTTGGACACTCCA TGT

GGTAGAGTGTCAGTTTGTCAAATACCCCAAGTGCGGCACATGCTTACCAGCTCTAGG CCA

GGGCAGATGGGATATGACGAATGGACTGCCAGCTGGATACAAGGATGCTCACCAAG CACC

AAGTTCTCACAAGTTATTTATGTGACTTTGCAGGAACTGAGGCATTATATCTGAGG ACA

CCAGGGGAAAAGTGTGGCATCTCAGGGAAATACAGCCCTGGGCTGTGTCTACACAC ACCA

TGAGAGTGCTGATGGGGGCGCAATAGTCTTGAAAATGTATAAAGTGTCCAGGAATG GAAG

TGCTCTTTGATTCATTATTATTTTCTTCCTTCATATTCCCCTCCCAGAGTCTCCTATCT
A
GGACATCAGCATTCTCACACAAGCCTAATGGCTTATCTGAGTAAGCAGGGCTTAGA
AATT
CACTTTCTTGATACTCAGTCTTGCCTTCTAAACACTCCTTGATCTTGCCTACCTCTCCC
C
TTTTCCACATGTCTTTTCCTGTAGGAACACTTTCTCCATTTATTCCTGCCTATCCAATT
C
TTCCCTATATTTCCTGGACCAGCTAAAGTCCAGTGTTTCCAGAGACTTTTGAAAGTC
AAC
TTACACTTTTTCCTTCTTCATTCACAAAGCTCTTCTTCCCTGGGCCCTGG

Hsa miR-455
ENST00000384993
>chromosome:GRCh37:9:116971114:116972409:1

TTATGAGAGCTGGGCTTTGAAGGATTTGGCTCAGAAGTCATGAGGGATGGGAAGGA
CATG
CCTGGTGGAGGGGACAGAGTGAGGAAAGGCTGGGAGGGAGGAAATGAGATGCATC
CTAGG
GAAGAGCCCCAGAATGGTGACGAGCCCCAGCTGTGGAGTCAGCTGGCCCTGGGTGA
CCCT
TGAACTGATTAGGAATTCCCGAAGAGAATTCACAGCTCCACTACTGACTAGTCGTGT
TGC
TTTATCTACAGAGCCTCAGTTTCTTTGTCTGTCCAGTGTGGGTGGTGACAATCTGGGG
GG
TCCTTGTGAGAGTCTAATGTGGTAGCATGCATGTGCCTGGCACAATGTTGGCACTCC
GGG
TTCGCAGCCGCTGTTAGTTAATGCCAGCACTCAGGCGGCCAGAGGTGGATGTAAGC
CCTA
CATCCAGGACCTTGAAGGCCTAGGAGGAGCCATGGCAGGAGCCACGGGCACCTACC
AGCA
TCCCTGGGGGTGGGCAGGGCTTGGTGCCGTGCTAGCATCTAACCCAGCCGCGAGCTT
CCT

TCTGCAGGTCCTGGAGCCCTGGCGTGGGGCGGGCCTCCTGCCGGCGAGCGCCTGCG
CCCT
TCCCTGGCGTGAGGGTATGTGCCTTTGGACTACATCGTGGAAGCCAGCACCATGCAG
TCC
ATGGGCATATACACTTGCCTCAAGGCCTATGTCATCGAGGAGCCACCGGAGCTGCC
ACTG
CCACCAGGGAGGAAGAGGAGGAGCCGGGATGTGGGATGGCAGTGGTGGGTGGGCT
GCGGC
AGGTTGGGCCAGCCACACCTCACTGCTTGACCGCTCTGACCCCCTTTCTTCTCTTTCC
TA
GGGCTACATTGGGCTCCCAGGGCTCTTCGGCCTGCCAGGGTCTGATGGAGAACGAG
TAAG
TTTGCTTCTTTGGTTATTCACCATCCACAGCCACCCCTGCCCAAACAGAGCAGAACC
TGC
CAGGCACCAGCCTGGGGAAGTCAAGGAATTCCTCCTGGAGGAGGTGCACTTGAGTC
GAGT
AGGAGTCTGATAAGGGGACAAGTCAAGGCAGATGCATTCCAGGCATAGGGAACAG
CATGT
GCGAAGTCAGGGATTTACGGTAACTTGGCGAGTTTAACACTCAACACTGCCAAAGT
GCTG
CGTGAGAGGGTGAGAGTGGTGGGAGTTGAGGCTGGAGAGGACCCTGGGGCAATGTC
TTAG
GTGCCCAGGACTAAGGTTAGAAAAGGAACTCTGTTCTGAGGGTATGCGGAGCCTCT
TGAG
GACTTGCAACCAATGAACACCGGGCCCACTTTATAA

Hsa miR-665
ENST00000390187
>chromosome: GRCh37:14:10140770:101342041:1

AGGCGTCCCCTCTGCCATCCGAGCGCTTGCACTGCGCTTCCGGGAGGAGGGTTGCGG
CCC
GTAGTCAGTAGTTGGGGGGTGGGAACGGCTTCATACAGGAGTTGATGCACAGTTAT
CCAG

CTCCTATATGATGCCTTTCTTCATCCCCTTCAACCACGCGCAGCCCCCGGACCCTCCT
CT
GCACCCTTGGCTGCACGGGGACGCGTCCTCAAGTCCGCTGCTGTCCTGCCAGCCTCT
TCC
CGGCATGCTCCTTGGGCTCTTCTCTCTCTTCTGCAGCTCTCTGACTTCCTGGAGGG
GA
GGGCCTGGGGCTGGGCCATCTCTGAGGCCCCTCCTTGGCAGGGAACTATGCCCCTGG
TGG
GACACCCCCTGGGCTGACTCCACAGCCAAGGGCAGATAGGCCGGAGGGGGAGTTGG
GTAG
CCCTGGGGCTTCTCCCACTGAGGGGCTGGGGCTGTGCTCCCACACAGAGCCCTCCAA
AGG
GACCCGGCCCACAGGATGGATGGACAGACGTTGGAGAGATGGTGCCCCTTTGTGCC
CAGG
GTGGAGGGGGTGGGGCTTTGGCGGACCAGGGCTGGCCCTTTCTGTGCCCCAAGAGG
AGGG
TCTCCTCGAGGGGTCTCTGCCTCTACCCAGGACTCTTTCATGACCAGGAGGCTGAGG
CCC
CTCACAGGCGGCTTCTTACTCTCTCCTTAAACCTGTTCTGGAGACATTTCCCCTCTCC
CC
AAGGATTCCCAACTCTGGGTCCCCAGAAGCTGTCAGCTGGGCTCCCTTCTTCCCCTG
TGC
GTACCCTGCTCAGGCTGGGTACCTGAGGGTGCCCAATGGCAACATGAGCAGGGGGC
ACGG
CCGTGGCCCCTGGTTCTTGGACCTCTGTCTTCTGCCTCTCTGACTAACTCAGGTCCTG
TG
CATGGCAGAGGTGACCCGCACCAAGCCCTGTGATATTGGCATCATTAACCCCATTAA
CAA
GCCACGACAGCCAGCCCAGGCATGAGCATCGGGGCAGCAGGGCAGCCAGTGTGGG
GAGAG
AGGCCTTGCAGTAGCTGAGGCCATTAGAGAGGACAAGAGGGTGCAGGAGGGAGCA
TCCTC

ACCTCTGCCCTTCCCACTTACATCCCATCTCCCCTTCCTCACCCCTCCTTCCCCATGC
AC
CCCTTCTTCCTGTAGGAGGGAGCACCCTCACCTCTGCCCTTCCCACTCACCTCCCATC
TT
CCCTTTCTCACCCCTCCTCCCCAATGCACCCCCTCCCTTCCTGCAGGAGGGAGCACCC
TC
TCCCCTGCCCTT

## Claims

1. Nucleic acid molecules comprising at least ten contiguous bases having a sequence as shown in the sequence of miR-223 (SEQ ID NO: 16) for treating fibrosis.

2. The nucleic acid molecule according to claim 1, wherein up to 10 % of the contiguous bases are non-complementary.

3. The nucleic acid molecule according to any one of claims 1 or 2, further comprising at least one base at the 5' end and/or at least one base at the 3' end.

4. The nucleic acid molecule according to any one of claims 1 to 3, wherein said nucleic acid molecule comprises the miR-223 precursor molecule having a sequence as shown in SEQ ID NO: 8 or a fragment thereof.

5. The nucleic acid molecule according to any one of claims 1 to 4, wherein said nucleic acid molecule consists of a molecule having a sequence as shown in SEQ ID NO: 16 (miR-223).

6. The nucleic acid molecule according to any one of claims 1 to 5, wherein said nucleic acid molecule consists of a molecule having the miR-223 precursor sequence as shown in SEQ ID NO: 8.

7. The nucleic acid molecule according to any one of claims 1 to 6, wherein said fibrosis is selected from the group consisting of cardiac fibrosis, pulmonary fibrosis, renal fibrosis, hepatic fibrosis, skeletal muscle fibrosis and radiation-induced fibrosis.

8. The nucleic acid molecule according to claim 7, wherein said fibrosis is cardiac fibrosis.

9. The nucleic acid molecule according to claim 8, wherein said cardiac fibrosis is a cardiac disease or disorder.

10. The nucleic acid molecule according to claim 9, wherein said cardiac disease is selected from the group consisting of heart failure, cardiac hypertrophy, heart-related storage disease, cardiomyopathy, constrictive pericarditis, coronary artery disease, acute myocardial infarction, chronic myocardial infarction, cardiac arrhytmia, myocarditis-related fibrosis and heart valve disease.

11. The nucleic acid molecule according to claim 10, wherein said heart failure is selected from the group consisting of hypertensive heart failure, diastolic heart failure, right heart failure and systolic heart failure.

Figure 1.

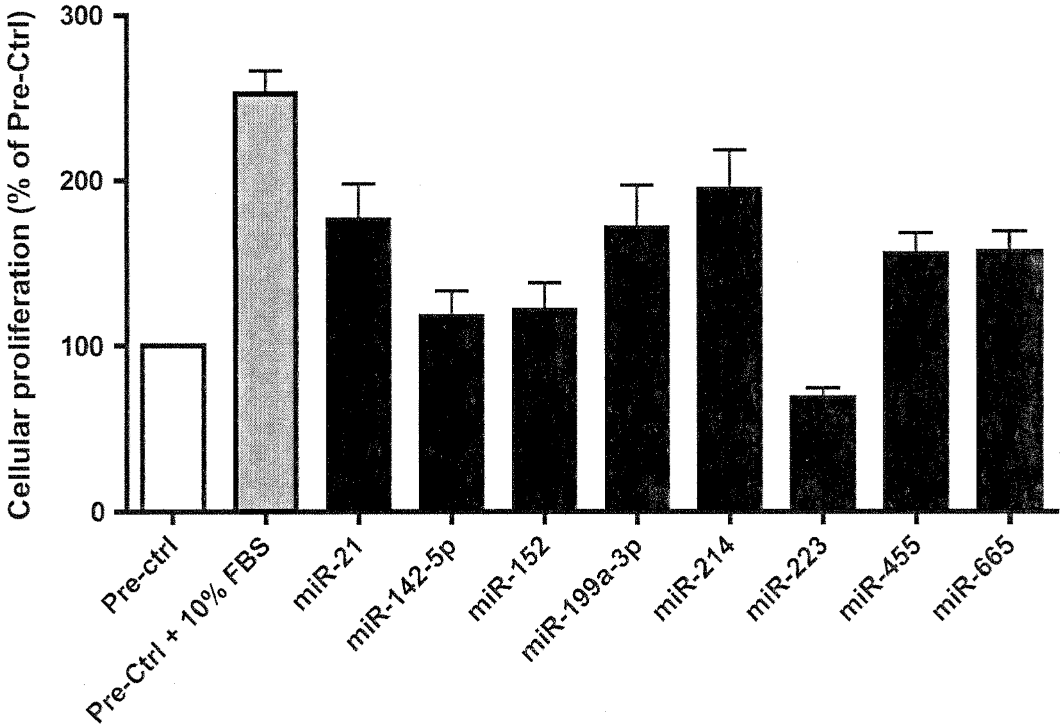
Cellular proliferation (% of Pre-Ctrl)
300
200
100
0
Pre-ctrl
Pre-Ctrl + 10% FBS
miR-21
miR-142-5p
miR-152
miR-199a-3p
miR-214
miR-223
miR-455
miR-665

Figure 2.

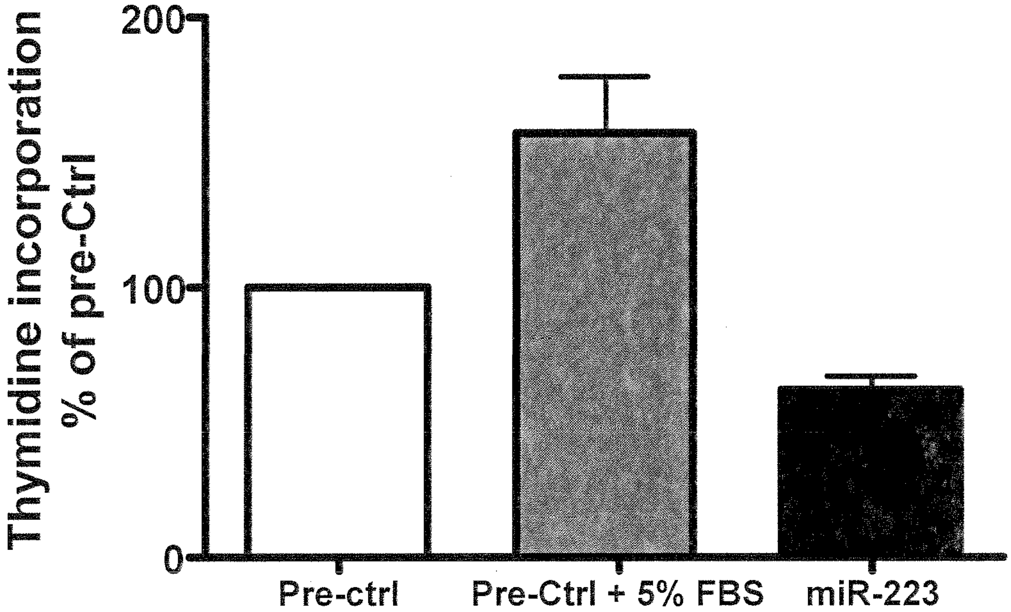
Thymidine incorporation
% of pre-Ctrl
200
100
0
Pre-ctrl
Pre-Ctrl + 5% FBS
miR-223

Figure 3.

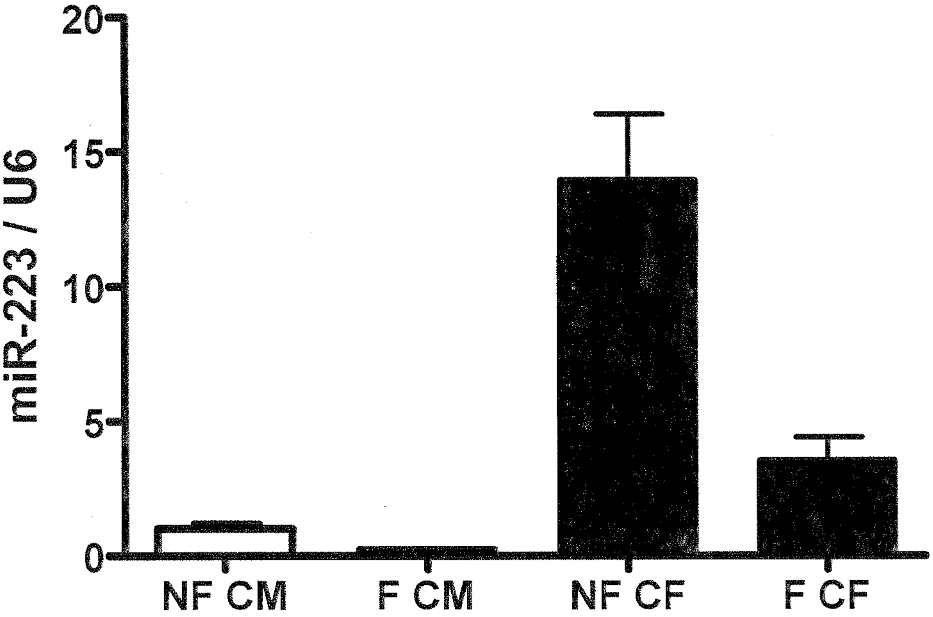

Figure 4.

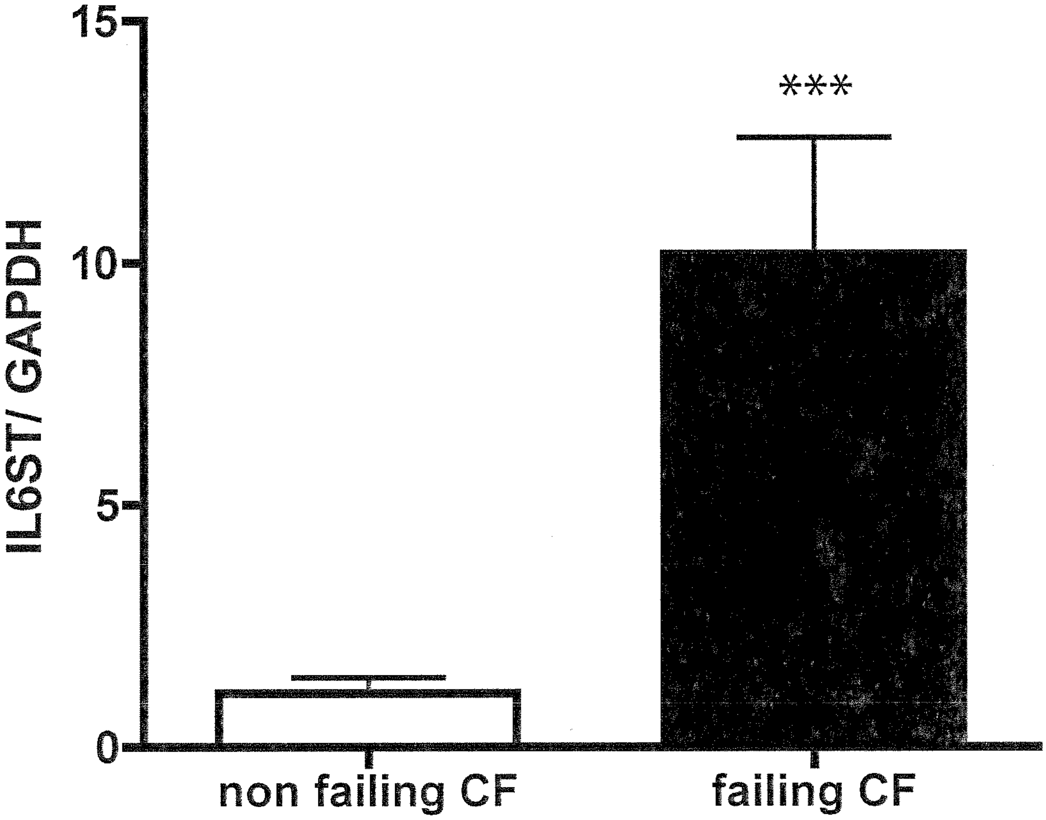

Figure 5.

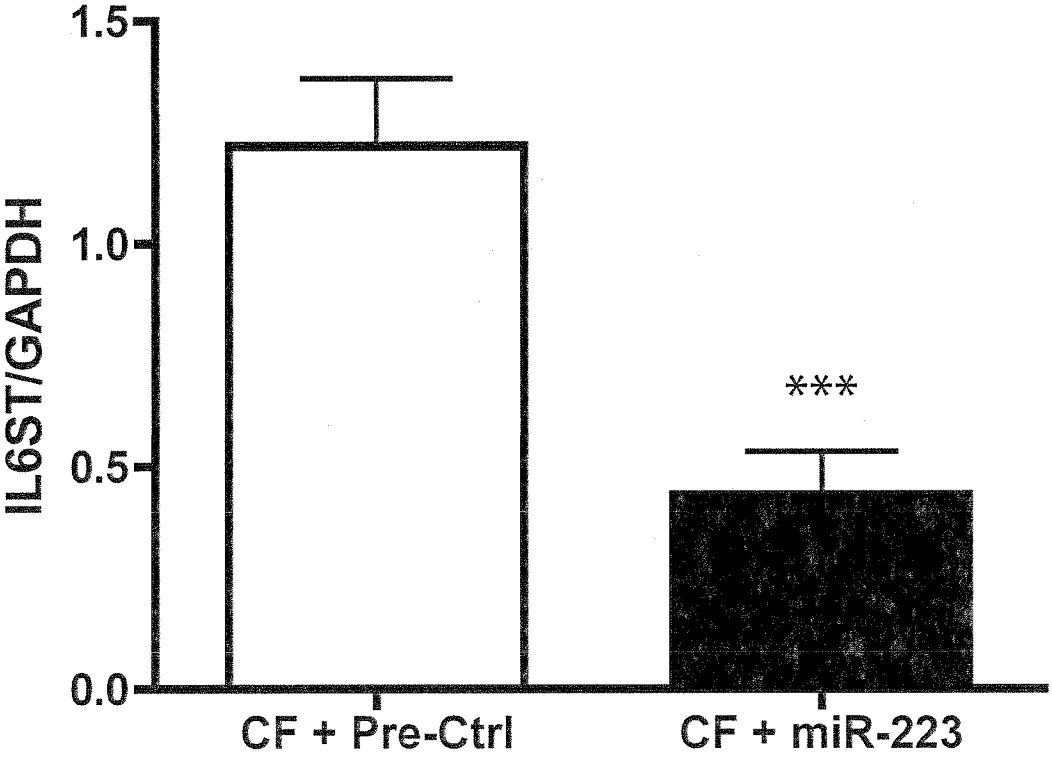

Figure 6.

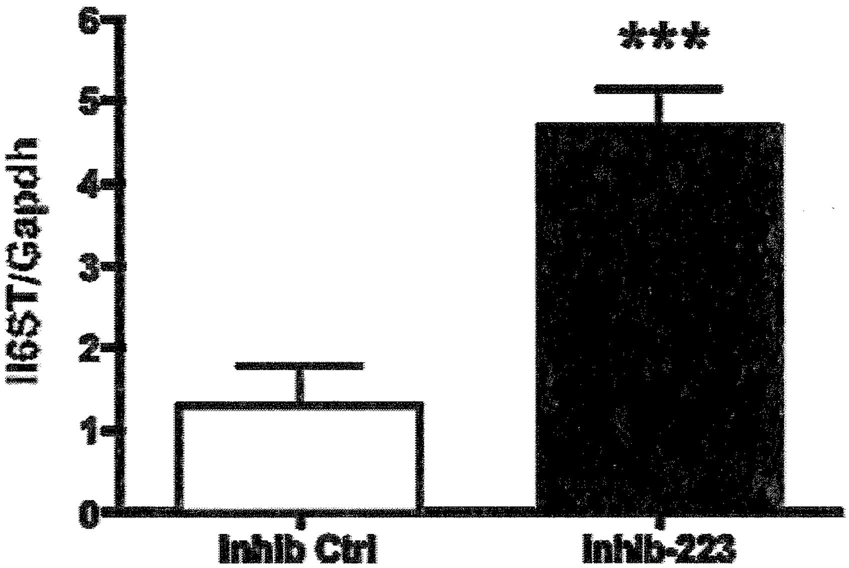

**Figure 7.**

| miRNA | Accession number | Sequence |
|---|---|---|
| miR-21 (SEQ ID NO: 1) | MI0000077 | UGUCGGGUAGCUUAUCAGACUGAUGUUGACUGUU GAAUCUCAUGGCAACACCAGUCGAUGGGCUGUCU GACA |
| miR- 142-5p (SEQ ID NO: 2) | MI0000458 | GACAGUGCAGUCACCCAUAAAGUAGAAAGCACUA CUAACAGCACUGGAGGGUGUAGUGUUUCCUACUU UAUGGAUGAGUGUACUGUG |
| miR-152 (SEQ ID NO: 3) | MI0000462 | UGUCCCCCCCGGCCCAGGUUCUGUGAUACACUCCG ACUCGGGCUCUGGAGCAGUCAGUGCAUGACAGAA CUUGGGCCCGGAAGGACC |
| miR-199a-1 (SEQ ID NO: 5) | MI0000242 | GCCAACCCAGUGUUCAGACUACCUGUUCAGGAGG CUCUCAAUGUGUACAGUAGUCUGCACAUUGGUUA GGC |
| miR-199a-2 (SEQ ID NO: 6) | MI0000281 | AGGAAGCUUCUGGAGAUCCUGCUCCGUCGCCCCA GUGUUCAGACUACCUGUUCAGGACAAUGCCGUUG UACAGUAGUCUGCACAUUGGUUAGACUGGGCAAG GGAGAGCA |
| miR-214 (SEQ ID NO: 7) | MI0000290 | GGCCUGGCUGGACAGAGUUGUCAUGUGUCUGCCU GUCUACACUUGCUGUGCAGAACAUCCGCUCACCU GUACAGCAGGCACAGACAGGCAGUCACAUGACAA CCCAGCCU |
| miR-223 (SEQ ID NO: 8) | MI0000300 | CCUGGCCUCCUGCAGUGCCACGCUCCGUGUAUUUG ACAAGCUGAGUUGGACACUCCAUGUGGUAGAGUG UCAGUUUGUCAAAUACCCCAAGUGCGGCACAUGC UUACCAG |
| miR-455 (SEQ ID NO: 9) | MI0003513 | UCCCUGGCGUGAGGGUAUGUGCCUUUGGACUACA UCGUGGAAGCCAGCACCAUGCAGUCCAUGGGCAU AUACACUUGCCUCAAGGCCUAUGUCAUC |
| miR-665 (SEQ ID NO: 10) | MI0005563 | UCUCCUCGAGGGGUCUCUGCCUCUACCCAGGACUC UUUCAUGACCAGGAGGCUGAGGCCCCUCACAGGC GGC |

**Figure 8.**

| miRNA | Accession number | Sequence |
|---|---|---|
| miR-21 (SEQ ID NO: 11) | MIMAT0000076 | UAGCUUAUCAGACUGAUGUUGA |
| miR-142-5p (SEQ ID NO: 12) | MIMAT0000433 | CAUAAAGUAGAAAGCACUACU |
| miR-152 (SEQ ID NO: 13) | MIMAT0000438 | UCAGUGCAUGACAGAACUUGG |
| miR-199a-3p (SEQ ID NO: 14) | MIMAT0000232 | ACAGUAGUCUGCACAUUGGUUA |
| miR-214 (SEQ ID NO: 15) | MIMAT0000271 | ACAGCAGGCACAGACAGGCAGU |
| miR-223 (SEQ ID NO: 16) | MIMAT0000280 | UGUCAGUUUGUCAAAUACCCCA |
| miR-455 (SEQ ID NO: 17) | MIMAT0004784 | GCAGUCCAUGGGCAUAUACAC |
| miR-665 (SEQ ID NO: 18) | MIMAT0004952 | ACCAGGAGGCUGAGGCCCCU |

Europäisches Patentamt
European Patent Office
Office européen des brevets

# EUROPEAN SEARCH REPORT

Application Number
EP 09 17 2129

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| E | WO 2009/137807 A2 (ASURAGEN INC [US]; SHEN JIKUI [US]; KELNAR KEVIN [US]; SHELTON JEFFREY) 12 November 2009 (2009-11-12)<br>* claims 16, 18 *<br>----- | 1-11 | INV.<br>C12N15/11<br>A61K31/7088<br>A61P19/04 |
| X | WONG Q W-L ET AL: "MicroRNA-223 Is Commonly Repressed in Hepatocellular Carcinoma and Potentiates Expression of Stathmin1"<br>GASTROENTEROLOGY, ELSEVIER, PHILADELPHIA, PA,<br>vol. 135, no. 1, 1 July 2008 (2008-07-01), pages 257-269, XP022823123<br>ISSN: 0016-5085<br>[retrieved on 2008-04-11]<br>* abstract; figure 4a *<br>----- | 1-4,7-11 | ADD.<br>A61P9/00 |
| X | VAN ROOIJ EVA ET AL: "Dysregulation of microRNAs after myocardial infarction reveals a role of miR-29 in cardiac fibrosis"<br>PROCEEDINGS OF THE NATIONAL ACADEMY OF SCIENCES OF THE UNITED STATES OF AMERICA,<br>vol. 105, no. 35, September 2008 (2008-09), pages 13027-13032, XP002568746<br>ISSN: 0027-8424<br>* abstract; figures 1c, 1d *<br>----- | 1-4,7-11 | TECHNICAL FIELDS SEARCHED (IPC)<br>C12N<br>A61K<br>A61P |

The present search report has been drawn up for all claims

1

| Place of search | Date of completion of the search | Examiner |
|---|---|---|
| The Hague | 1 March 2010 | Vreugde, Sarah |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons
& : member of the same patent family, corresponding document

EPO FORM 1503 03.82 (P04C01)

## ANNEX TO THE EUROPEAN SEARCH REPORT ON EUROPEAN PATENT APPLICATION NO. EP 09 17 2129

This annex lists the patent family members relating to the patent documents cited in the above-mentioned European search report.
The members are as contained in the European Patent Office EDP file on
The European Patent Office is in no way liable for these particulars which are merely given for the purpose of information.

01-03-2010

| Patent document cited in search report | Publication date | Patent family member(s) | Publication date |
|---|---|---|---|
| WO 2009137807 A2 | 12-11-2009 | US 2009281167 A1 | 12-11-2009 |

EPO FORM P0459

For more details about this annex : see Official Journal of the European Patent Office, No. 12/82

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- US 3773919 A
- EP 58481 A
- EP 133988 A
- DE 3218121
- EP 52322 A
- EP 36676 A
- EP 88046 A
- EP 143949 A
- EP 142641 A
- JP 58118008 A
- US 4485045 A
- US 4544545 A
- EP 102324 A


### Non-patent literature cited in the description


- **Bartel.** *Cell,* 2004, vol. 116, 281-297
- **Chen et al.** *Science,* 2004, vol. 3033, 83-86
- **Ledley.** *Pharmaceutical Research,* 1996, vol. 13, 1595-1614
- **Verma et al.** *Nature,* 1997, vol. 387, 239-242
- **Sambrook ; Russell.** Molecular Cloning, A Laboratory Manual. Cold Spring Harbor Laboratory, 2001
- **Ausubel.** Current Protocols in Molecular Biology. Green Publishing Associates and Wiley Interscience, 1989
- **Sambrook.** loc. cit. 2001
- **Ausubel.** loc. cit. 1989
- Nucleic acid hybridization, a practical approach. IRL Press Oxford, 1985
- **Thompson.** *Nucl. Acids Res.,* 1994, vol. 2, 4673-4680
- **Brutlag Comp.** *App. Biosci.,* 1990, vol. 6, 237-245
- **Altschul.** *Nucl. Acids Res.,* 1997, vol. 25, 3389-3402
- **Altschul.** *J. Mol. Evol.,* 1993, vol. 36, 290-300
- **Altschul.** *J. Mol. Biol.,* 1990, vol. 215, 403-410
- **Henikoff.** *PNAS,* 1989, vol. 89, 10915
- **Altschul.** *loc. cit.,* 1997
- **Altschul.** *loc. cit.,* 1993
- **Altschul.** *loc. cit.,* 1990
- **Brutlag.** *Comp. App. Biosci.,* 1990, vol. 6, 237-245
- **Kurreck.** *Eur. J. Biochem.,* 2003, vol. 270, 1628-1644
- **Eckstein.** *Antisense Nucleic Acids Drug Dev.,* 2000, vol. 10, 117-121
- **Gryaznov et al.** *J. Am. Chem. Soc.,* 1994, vol. 116, 3143-3144
- **Kurreck et al.** *Nucleic Acids Res.,* 2002, vol. 30, 1911-1918
- **Elayadi et al.** *Curr. Opinion Invest. Drugs,* 2001, vol. 2, 558-561
- **Orum et al.** *Curr. Opinion Mol. Ther.,* 2001, vol. 3, 239-243
- **Koshkin et al.** *Tetrahedron,* 1998, vol. 54, 3607-3630
- **Obika et al.** *Tetrahedron Lett.,* 1998, vol. 39, 5401-5404
- **Damha et al.** *J. Am. Chem. Soc.,* 1998, vol. 120, 12976-12977
- **Nielson.** *Methods Enzymol.,* 1999, vol. 313, 156-164
- **Elayadi et al.** *id*
- **Braasch et al.** *Biochemistry,* 2002, vol. 41, 4503-4509
- **Nielsen et al.** *Science,* 1991, vol. 254, 1497-1500
- **Nielsen et al.** Peptide Nucleic Acids-Protocols and Applications. Horizon Scientific Press, 1-19
- **Nielsen et al.** *Science,* vol. 254, 1497-1500
- **Heasman.** *Dev. Biol.,* 2002, vol. 243, 209-214
- **Wang et al.** *J. Am. Chem. Soc.,* 2000, vol. 122, 8595-8602
- **Verbeure et al.** *Nucleic Acids Res.,* 2001, vol. 29, 4941-4947
- **Steffens et al.** *J. Am. Chem. Soc.,* 1997, vol. 119, 11548-11549
- **Renneberg et al.** *J. Am. Chem. Soc.,* 2002, vol. 124, 5993-6002
- **Wang et al.** *Proc. Natl. Acad. Sci. USA,* 1999, vol. 96, 13989-13994
- **Liang et al.** *Eur. J. Biochem.,* 2002, vol. 269, 5753-5758
- **Lok et al.** *Biochemistry,* 2002, vol. 41, 3457-3467
- **Damha et al.** *J. Am. Chem. Soc.,* 2002, vol. 120, 12976-12977

- **Sidman, U. et al.** *Biopolymers,* 1983, vol. 22, 547-556
- **R. Langer et al.** *J. Biomed. Mater. Res.,* 1981, vol. 15, 167-277
- **R. Langer.** *Chem. Tech.,* 1982, vol. 12, 98-105
- **R. Langer et al.** *Id.*
- **Epstein et al.** *Proc. Natl. Acad. Sci. (USA),* 1985, vol. 82, 3688-3692
- **Hwang et al.** *Proc. Natl. Acad. Sci. (USA),* 1980, vol. 77, 4030-4034
- **Hughes et al.** *Drug Discovery Today,* 2001, vol. 6, 303-315
- **Becker et al.** In Antisense Technology in the Central Nervous System. Oxford University Press, 147-157
- **Engelhardt S ; Hein L ; Wiesmann F ; Lohse MJ.** *Progressive hypertrophy and heart failure in betal-adrenergic receptor transgenic mice,* 08 June 1999, vol. 96, 7059-7064